(19) Europäisches Patentamt
European Patent Office
Office européen des brevets

(11) **EP 2 460 825 B1**

(12) **EUROPEAN PATENT SPECIFICATION**

(45) Date of publication and mention
of the grant of the patent:
**22.08.2018 Bulletin 2018/34**

(51) Int Cl.:
**A61K 38/00** [(2006.01)]

(21) Application number: **10803913.2**

(22) Date of filing: **29.07.2010**

(86) International application number:
**PCT/CN2010/075548**

(87) International publication number:
**WO 2011/012080 (03.02.2011 Gazette 2011/05)**

(54) **DERIVATIVE OF GLP-1 ANALOGUE OR ITS PHARMACEUTICAL SALTS AND THEIR USE**

DERIVAT AUS EINEM GLP-1-ANALOG ODER DESSEN PHARMAZEUTISCHEN SALZEN UND SEINE VERWENDUNG

DÉRIVÉ DE L'ANALOGUE DU GLP-1 OU SES SELS PHARMACEUTIQUES ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **30.07.2009 CN 200910165559**

(43) Date of publication of application:
**06.06.2012 Bulletin 2012/23**

(73) Proprietor: **Jiangsu Hansoh Pharmaceutical Group Co., Ltd.**
**Jiangsu 222047 (CN)**

(72) Inventors:
• **WANG, Yali**
**Lianyungang**
**Jiangsu 222047 (CN)**
• **LÜ, Aifeng**
**Lianyungang**
**Jiangsu 222047 (CN)**
• **SUN, Changan**
**Lianyungang**
**Jiangsu 222047 (CN)**
• **YUAN, Hengli**
**Lianyungang**
**Jiangsu 222047 (CN)**

(74) Representative: **EP&C**
**P.O. Box 3241**
**2280 GE Rijswijk (NL)**

(56) References cited:
| | |
|---|---|
| WO-A1-01/04156 | WO-A1-2008/058461 |
| WO-A2-00/41548 | WO-A2-2007/124461 |
| CN-A- 1 232 470 | CN-A- 101 128 214 |
| US-A- 5 424 286 | US-A- 5 545 618 |
| US-A1- 2003 199 672 | US-A1- 2006 069 029 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

This invention relates to a series of derivatives of the human Glucagon-like peptide-1 (GLP-1) analogue and pharmaceutical salts thereof. The derivatives of the GLP-1 analogue provided in this invention have the function of the human GLP-1 and a longer half-life *in vivo* compared with the human GLP-1. The present invention also relates to the use of the derivatives of the GLP-1 analogue, the pharmaceutical salts thereof, or pharmaceutical compositions containing the derivatives of the GLP-1 analogue or the pharmaceutical salts thereof in the treatment of non-insulin-dependent diabetes, insulin-dependent diabetes or obesity.

### BACKGROUND OF THE INVENTION

Diabetes mellitus is a global epidemic disease and is a syndrome of the metabolic disorders of glucose, protein and lipid due to the absolute or relative deficiency of insulin (Chen Ruijie. Status of research on diabetes drugs, Academic journal of Guangdong College of Pharmacy, 2001, 7(2):131-133). The Diabetes mellitus can be divided into type I diabetes mellitus and type II diabetes mellitus (Type 2 diabetes mellitus, T2DM, the same below) according to the pathogenesis thereof. 90-95% of all the patients diagnosed with diabetes mellitus suffer from T2DM, and the patients are often accompanied with obesity, a deficiency of physical activity (physical inactivity), an older age, a family history of diabetes mellitus, a damage in glucose metabolism and having a family history of diabetes mellitus and the like. T2DM is also a progressive disease. According to the statistical data in 2000, World Health Organization estimated that there are about 171 million people world widely who suffer from diabetes mellitus; in 2005, the Centers for Disease Control and Prevention of U.S.A. estimated that there are 20.8 million Americans who suffer from diabetes mellitus, which is about 7% of the population of the United States of America; in 2006, according to the statistics of the International Diabetes Federation, the global number of the patients suffering from diabetes mellitus is about 246 million (about 5.9% of the totally global population) and furthermore 46% of the patients are 40-59 years old.

Studies have shown that there presents very important difference on how to respond to the glucose between health ones and the patients with T2DM. The response to the postprandial hyperglycemia of health ones belongs to the early insulin response.

T2DM is characterized by the inhibition of the secretion of the insulin and the pancreatic β-cell dysfunction, resulting in an insulin deficiency and a hyperglycemia (Ferrannini E. Insulin resistance versus insulin deficiency in non-insulin-dependent diabetes mellitus: problems and prospects. Endocr Rev. 1998, 19(4):477-490). T2DM patients typically suffer from a postprandial and fasting hyperglycemia (fasting glucose> 125mg/dL), and the high blood sugar is mainly due to that pancreatic β-cells fail to secrete enough insulin to compensate insulin deficiency caused by the inhibition of insulin in the surrounding tissue (Weyer C., Bogardus C., Mott DM., et al. The natural history of insulin secretory dysfunction and insulin resistance in the pathogenesis of type 2 diabetes mellitus. J. Clin. Invest. 1999, 104(6): 787-794).

The one major risk factor of T2DM is obesity, which are very harmful to the human health. The risk suffering from a cardiovascular disease and an abnormal death of the patients increases, meanwhile T2DM often co-exists with other high-risk diseases such as hypertension, dyslipidemia and obesity; 60% of the T2DM patients are accompanied by microvascular complications, including retinopathy and neuropathy, and also are accompanied by cardiovascular morbidities associated with T2DM such as the coronary heart disease, myocardial infarction and shock and the like. In U.S.A., cardiovascular diseases (CVD) is the major cause resulting in morbidity and death, and T2DM is the major risk factor causing macrovascular complications such as an atherosclerosis, myocardial infarction, shock and peripheral vascular diseases. The death risk caused by heart diseases and a shock of an adult with diabetes is 2-4 times higher than that of a non-diabetes person. In addition, nearly 65% of people with diabetes die of heart diseases and the shock.

In addition to the physical and physiological harm to patients, T2DM causes great economic burden on the society. According to statistics, the cost of the treatment of complications associated with diabetes is about $ 22.9 billion, the total cost of the treatment of T2DM and complications thereof is nearly $ 57.1 billion, and the total cost not included in the budget is more than $ 8 billion every year in the United States.

Drugs for the treatment of T2DM have been the focus, such as the early oral hypoglycemic drugs of sulfonyl class and biguanide class and the recent insulin sensitizer and α-glucosidase inhibitors, the development of animal insulins and human insulins and a variety of new techniques of formulations, the research of new mechanisms of drug treatment by simply increasing insulin to new ways acting on the insulin-producing. Weight gain is the common side-reaction after the administration of oral or injection hypoglycemic agents, which may reduce compliance, and may increase the risk of developing cardiovascular disease. Therefore, developing new types of drugs for the treatment of T2DM which have high safety, good patient compliance and low side-reaction becomes the hot topic of the research institutions and pharmaceutical companies against the diabetes.

As early as 100 years ago, Moore has proposed that the duodenum can secrete a "chemical stimulant" which can stimulate the secretion in pancreas and has tried to inject gut-extract to treat diabetes. Subsequently it has been discovered that humoral factors derived from intestinal secretion can enhance the function of the pancreas endocrine, and about 50% of insulin secretion induced by intravenous or oral glucose is derived from the stimulus of peptides produced by the gut. Therefore Zunz and Labarre created the concept of "incretin ". Two kinds of incretins have been isolated so far, namely glucose-dependent insulinotropic polypeptide (GIP) and glucagon-like peptide-1(GLP-1). Both of GIP and GLP-1 are secreted by the specific intestinal nerve cells when the nutrient is absorbed, in which GIP is secreted by the duodenum and proximal jejunal K cells, GLP-1 is synthesized in L cells and mainly exist in the distal small bowel and colon (Drucker DJ. Enhancing incretin action for the treatment of type 2 diabetes. Diabetes Care. 2003, 26(10):2929-2940).

GLP-1 exists in two bio-active forms, namely GLP-1 (7-37) and GLP-1 (7-36) amide in the plasma, between which only one amino acid is different, and their biological effects and *in vivo* half-life are same (Drucker DJ. Enhancing incretin action for the treatment of type 2 diabetes. Diabetes Care. 2003, 26(10):2929-2940).

GLP-1 usually referred to is the common name of GLP-1 (7-37) and GLP-1 (7-36) amide. GIP and GLP-1 will be degraded to inactive forms by dipeptidyl peptidase-IV(DPP-IV) quickly after released in the gastrointestinal tract, so that the *in vivo* half-life of GIP and GLP-1 is very short (*in vivo* half-life of GIP is about 5-7min, *in vivo* half-life of GLP-1 is about 2min) (Drucker DJ. Enhancing incretin action for the treatment of type 2 diabetes. Diabetes Care. 2003, 26(10):2929-2940). Researches show that most of the degradation process occurs when the GIP and GLP-1 enter into the blood vessels containing DPP-IV, and a small amount of GLP-1 and GIP which have not been degraded will enter into pancreas and be bound with their binding sites to stimulate β-cells to release the insulin. Different from the mechanism of sulfonylurea to directly promote functional β-cells to release insulin, most of the effect of incretin are glucose-dependent. In addition, some *in vitro* tests on animals and human showed that GLP-1 also has functions such as α-cell suppression and reducing glucagon hypersecretion and so on.

Although plasma GIP levels in patients with T2DM are normal when the function of incretin declines or losses significantly, the GLP-1 levels in patients with T2DM decline, so the drugs based on GLP-1 contribute more to the treatment of T2DM. Although the levels of both GLP-1 (7-37) and GLP-1 (7-36) amide will increase in several minutes after a meal, the content of GLP-1 (7-36) amide is more, so the GLP-1 secretion might have been greatly increased by the double effect of endocrine and transmission of neural signal before the digested food enters the small intestine and colon from the bottom of digestive tract. The plasma level of GLP-1 under fasting state is very low (about 5-10pmol/L), and is increased rapidly after eating (up to 15-50pmol / L). Under the double function of DPP-IV and renal clearance, the level *in vivo* of GLP-1 in circulating is decreased rapidly, while the work to research whether other enzymes such as human neutral endopeptidase 24 • 11 and the like also play a vital role in inactivating the activity of GLP-1 is in progress, because the second amino acid residue of GLP-1 is an alanine which is a good substrate of DPP-IV so the GLP-1 is easy to be degraded into inactive peptide fragments. In fact, the DPP-IV *in vivo* is the key reason for the loss of the activity of the incretin. Experiments show that GLP-1 level in mice, in which DPP-IV gene has been silenced, is higher than in normal mice significantly, and the insulin secretion is increased too. Just because the present of DPP-IV, the content *in vivo* of the complete and biologically active GLP-1 is only 10-20% of the total content of GLP-1 in plasma (Deacon CF, Nauck MA, Toft-Nielsen M, et al. Both subcutaneously and intravenously administered glucagon-like peptide 1 are rapidly degraded from the NH2-terminus in type 2-diabetic patients and in healthy subjects. Diabetes. 1995, 44(9): 1126-1131).

GLP-1 and GIP play their respective roles through binding to G-protein-coupled receptors (GPCRs) which have completely different structures. Most of GIP receptors are expressed by the pancreatic β-cells, and a minor part of GIP receptors are expressed by the adipose tissue and the central nervous system. In contrast, GLP-1 receptors are mainly expressed in the pancreatic α- and β- cells and peripheral tissues including the central and peripheral nervous systems, brain, kidney, lung and gastrointestinal tract and the like. The activation of two incretins in β-cells will result in the rapid increase of the level of cAMP and intracellular calcium in the cells, thereby leading their secretion to the extracellular region in glucose-dependent manner. The sustained signal transmission from incretin receptors is associated with protein kinase A, resulting in the gene transcription, increasing insulin biosynthesis and stimulating β-cell proliferation(Gallwitz B. Glucagon-like peptide-1-based therapies for the treatment of type 2 diabetes mellitus. Treat Endocrinol. 2005, 4(6):361-370). The activation of GLP-1 receptor and GIP receptor can also inhibit the apoptosis of pancreatic β-cells of rodent and human, meanwhile increase their survival(Li Y, Hansotia T, Yusta B, et al. Glucagon-like peptide-1 receptor signaling modulates beta cell apoptosis. J Biol Chem. 2003, 278(1): 471-478). In consistence with the expression of GLP-1 receptor, GLP-1 can also inhibit glucagon secretion, gastric emptying and food intake, and enhance the degradation of glucose through the neural mechanism. It shall be noted that, same as other insulin secretion reaction, the role of GLP-1 to control the level of glucose is glucagon-dependent, while the role of glucagon of counter-regulatory release of glucagon caused by low blood sugar has been fully retained even at the pharmacological level of GLP-1.

The important physiological role of endogenous GLP-1 and GIP in glucose homeostasis has been studied in-depth through using receptor antagonists or gene knockout mice. Acute antagonism of GLP-1 or GIP reduces insulin secretion *in vivo* of rodent and increases plasma glucose content. Similarly, the mutant mice, in which GIP or GLP-1 receptor is inactivated, also experienced defective glucose-stimulated insulin secretion and damaged glucose tolerance. GLP-1 also has a function of regulating fasting blood glucose, because the acute antagonists or the damage on the GLP-1 gene will cause the increase of fasting glucose level of rodents; at the same time, GLP-1 is the basis of glucose control in human bodies and studies on the antagonist of Exendin (9-39) have shown that the destruction of GLP-1 funtion will result in defective glucose-stimulated insulin secretion, decreased glucose clearance rate, increased glucagon levels and accelerated gastric emptying. The physiological roles of GLP-1(Deacon CF. Therapeutic strategies based on glucagon-like peptide 1. Diabetes. 2004, 53(9):2181-2189) comprise: (1) helping to organize glucose absorption, mediate glucose-dependent insulin secretion; (2) inhibiting postprandial glucagon secretion, reducing hepatic glucose release; (3) regulating gastric emptying, preventing excessive circulating of glucose when the food is absorbed in the intestinal; (4) inhibiting food intake (such as appetite). Animal studies also showed a physiological role of GLP-1 stabilizing the number of pancreatic $\beta$-cells *in vivo.*

Due to the good effects of GLP-1 and GIP in controlling blood sugar and many other aspects, especially their characteristics not producing hypoglycemia and delaying gastric emptying to control weight attract the interest of many scientists. People try to research drugs based on GLP-1 and GIP for the treatment of T2DM. It is well known that T2DM patients lack or lose the incretin effect, wherein one reason is that incretin effect of GIP *in vivo* in the T2DM patient is significantly reduced; meanwhile, the level of GLP-1 *in vivo* in T2DM patient is very low, and the level of GLP-1 caused by dietary stimuli is significantly reduced (Toft-Nielsen MB, Damholt MB, Madsbad S, et al. Determinants of the impaired secretion of glucagon-like peptide-1 in type 2 diabetic patients. J Clin Endocrinol Metab. 2001, 86(8):3717-3723). Because the role of GLP-1 *in vivo* in patients with T2DM has been partially reserved, GLP-1 synergist is one of the research directions of the drugs designed to enhance the incretin effect in T2DM patients.

GLP-1 analogue, as same as endogenous GLP or GIP, can inhibit the release of glucagon *in vivo* and stimulate insulin secretion *in vivo* in the glucose-dependent manner. In addition, GLP-1 analogues have a role in the following symptoms: (1) low blood sugar. Different from other secretagogues drugs, GLP-1 analogues promote insulin secretion *in vivo* in a glucose-dependent manner and thus its role for lowering blood glucose exhibits a self-limitation, which generally does not cause hypoglycemia in large doses. Despite it has been reported in the literature that GLP-1 can reduce blood sugar to a level below normal level, this effect is transient and is considered as a natural result of GLP-1 promoting insulin secretion. Because the inactivation of insulin will take some time, when the stimulation effect of GLP-1 is reduced due to the decrease of the blood sugar level while there is not new insulin secretion, the original insulin still works. In a word, GLP-1 can temporarily reduce blood sugar to a level below normal level but does not cause serious and persistent hypoglycemia; the role on satiation and body weight. Besides the directly reducing blood glucose, GLP-1 can also reduce the quantity of food intake, which has been verified in rodents and human. Therefore the level of blood glucose can be controlled by reducing the body weight indirectly. GLP-1 also has the potential role of inhibiting the secretion of gastrin and gastric acid stimulated by eating, and these funtions show that GLP-1 may also have a role in the prevention of peptic ulcer. Action mechanisms of GLP-1 make itself become not only an ideal drug for the treatment of patients with type 2 diabetes, but also the drug for the treatment of patients with obesity diabetes. GLP-1 can enhance the satiety of the patients, reduce food intake and maintain body weight or lose weight; (3)maintaining the health of $\beta$-cells. Several studies suggest that GLP-1 can prevent the conversion from impaired glucose tolerance to diabetes, and some literatures report that GLP-1 class of compounds have direct effect on the growth and proliferation of pancreatic $\beta$-cells of experimental animals, and it was found by some experiments that GLP-1 can promote the differentiation from pancreatic stem cells to functional $\beta$-cells. These results suggest that GLP-1 has the function of protecting pancreatic islet and delaying the progression of diabetes, and can maintain the morphologies and functions of $\beta$-cells, while reduce the apoptosis of $\beta$-cells; (4) the effect on the postprandial hyperglycemia. This phenomenon represents a new direction for the treatment of T2DM. Because some oral drugs and exogenous insulins can not inhibit or reduce the exorbitant glucagon secretion in patients with T2DM, GLP-1 analogues can affect glucagon hypersecretion through directly inhibiting glucagon release or paracrine inhibition of glucagon resulted from promoting insulin secretion. The postprandial hyperglycemia can be reduced effectively through these two mechanisms; meanwhile the maintaining of the function of $\beta$-cells may also play a role in controlling the long-term postprandial hyperglycemia.

Meanwhile GLP-1 analogues are administered through subcutaneous injection, which doesn't need calculating the amount of carbohydrates to estimate the optimal drug dosage, and does not need self-monitoring the blood glucose, resulting that these kinds of drugs are easier to be used than the insulin.

A variety of effects of natural GLP-1 have been confirmed, which bring new hope for the treatment of T2DM, however, the natural human GLP-1 is very unstable and can be degraded by dipeptidyl peptidase IV (DPP-IV) and its half-life is only 1 ~ 2minutes. When using natural GLP-1 to lower blood sugar, continuous intravenous infusion or continuous subcutaneous injection is needed, resulting the poor clinical feasibility. Faced with this situation, researchers continue to explore the method to extend the action time of GLP-1. Therefore, the development of long-acting GLP-1 analogues or derivatives thereof becomes an important area of interest in the pharmaceutical field.

Exenatide is a synthetic Exendin-4, which is developed by the Eli Lilly Company and Amylin Company with trade name Byetta®. Exenatide has been approved for the treatment of T2DM by FDA and EMEA. It has 50% homology with mammalian GLP-1 in sequence and has a similar affinity site of the receptor with GLP-1(Drucker DJ, Nauck MA. The incretin system: glucagon-like peptide-1 receptor agonists and dipeptidyl peptidase-4 inhibitors in type 2 diabetes. Lancet. 2006, 368(9548):1696-1705), and it is encoded by the lizard-specific gene; compared with GLP-1, the position 2 Alanine in GLP-1 is replaced with a glycine in Exenatide, which effectively inhibits the enzymolysis of DPP-IV enzyme, and its half-life *in vivo* is about 60-90min (Kolterman OG, Kim DD, Shen L, et al. Pharmacokinetics, pharmacodynamics, and safety of exenatide in patients with type 2 diabetes mellitus. Am Health Syst Pharm. 2005, 62(2): 173-181), the *in vivo* concentration of Exenatide after a single subcutaneous injection is increased persistently and can arrive to the maximum plasma concentration after 2h or so, which can be maintained for 4-6h(Nielsen LL, Baron AD. Pharmacology of exenatide (synthetic exendin-4) for the treatment of type 2 diabetes. Curr Opin Investig Drugs. 2003, 4(4):401-05). It should be noted that the metabolism of Exenatide does not occur in the liver, but is degraded mainly by protein protease after filtered by renal glomeruli.

Exenatide has special glucose-regulating activities, including glucose-dependent enhance of insulin secretion, glucose-dependent inhibition of wrong excessive glucagon secretion, slowing gastric emptying and decreasing food intake and the like. Studies *in vitro* and *in vivo* in the models of diabetes found that Exenatide also has the effects of storing the first stage (first-phase) insulin secretion, promoting the proliferation of β-cell and promoting the regeneration of insulin from its precursor cell.

In order to achieve better control of blood glucose, twice a day injection of Exenatide is needed, which gives the patients a big inconvenience. Furthermore, Exenatide also has a mild to moderate nausea (about 40% of patients will have this reaction), diarrhea and vomiting (less than 15% of patients have both reactions); about 50% of Exenatide-treated patients can generate antibodies, although these antibodies do not affect the efficacy or lead to other clinical effects. Recently it is found that six patients occurred hemorrhage or symptoms of necrotizing pancreatitis after taking Byetta.

CJC-1131 is a GLP-1 analogue with peptidase resistance developed by ConjuChem Biotechnologies Inc., in which Ala in the position 2 of GLP-1 is replaced with D-Ala in order to enhance the ability of resisting the DPP-IV enzymolysis, and of which the structure contains an active reactive linker that can binds to serum albumin through covalent (non-reversible) manner(Kim JG, Baggio LL, Bridon DP, et al. Development and characterization of a glucagon-like peptide-1 albumin conjugate: the ability to activate the glucagon-like peptide 1 receptor in vivo. Diabetes 2003, 52(3):751-759), and the GLP-1-serum albumin complex retains the activity of GLP-1, while increases the stability to DPP-IV enzymolysis, extends action duration *in vivo,* and half-life in plasma thereof is about 20 days.

A study has found that the Ki was approximate 12nM (the Ki of GLP-1 is 5.2nM) when CJC-1131-serum albumin complex is bound to Chinese hamster ovary cell transfected with recombinant human pancreatic GLP-1 receptor; meanwhile the $EC_{50}$ of the complex activating cAMP is 11-13nM, wherein the $EC_{50}$ is similar to GLP-1's. Existing literatures show that this binding molecule can reduce postprandial blood glucose level of the mice whose blood sugar is normal or high, and tests show that this activity of CJC-1131 acts on a certain functional receptor of GLP-1, meanwhile in mice CJC-1131 also has an effect on slowing gastric emptying and inhibiting food intake and the like.

Part of the Phase II clinical trial of CJC-1131 has been completed. In September 2005, ConjuChem concluded that CJC-1131 may not be suitable for chronic dosing regimens after analysis of the existing test results, and suspended the clinical study of CJC-1131. The clinical trial of CJC-1131 hasn't been restarted yet.

Albugon (albumin-GLP-1) is a long-acting drug for the treatment of T2DM developed by GlaxoSmithKline authorized by Human Genome Sciences Inc., which is a fusion protein of GLP-1 (with mutations increasing the resistance to DDP-IV) and albumin. Its half-life in monkeys is 3 days. The basic idea of the development thereof is to couple the recombinant GLP-1 and serum albumin to form a complex; thereby its *in vivo* half-life is significantly increased. The administration of Albugon effectively reduces blood glucose level of mice, increases insulin secretion, slows gastric emptying and reduces food intake etc. (Baggio LL, Huang Q, Brown TJ, et al. A Recombinant Human Glucagon-Like Peptide (GLP)-1-Albumin Protein (Albugon) Mimics Peptidergic Activation of GLP-1Receptor-Dependent Pathways Coupled With Satiety, Gastrointestinal Motility, and Glucose Homeostasis. Diabetes 2004, 53(9):2492-2500). Currently Albugon is in phase III clinical trial.

WO9808871 discloses a GLP-1 derivative which is obtained through the modification on GLP-1(7-37) with fatty acid and the half life *in vivo* of GLP-1 is significantly enhanced. WO9943705 discloses a derivative of GLP-1, which is chemically modified on the N terminal, but some literatures report that modification of the amino acids on the Nterminal will significantly decrease the activity of the entire GLP-1 derivative (J. Med. Chem. 2000, 43, 1664 1669). In addition, CN200680006362, CN200680006474, WO2007113205, CN200480004658, CN200810152147 and WO2006097538 etc also disclose a series of GLP-1 analogues or derivatives thereof produced by chemical modification or amino acid substitution, in which the most representative one is liraglutide developed by Novo Nordisk, the phase III clinical trial of which has been finished. Liraglutide is a derivative of GLP-1, whose structure contains a GLP-1 analogue of which the sequence is 97% homologous with human GLP-1, and this GLP-1 analogue is linked with palmitic acid covalently to form Liraglutide, wherein the palmitic acid of the structure of Liraglutide is linked to serum albumin non-covalently, and this structural characteristics determines that it can be slowly released from the injection site without changing the activity of GLP -1 and the *in vivo* half life is extended; meanwhile the palmitic acid in the structure will form a certain steric hindrance to prevent the degradation by DPP-IV and to reduce renal clearance. Because of the characteristics described above, the half-life of Liraglutide in the human body administered by subcutaneous injection is about 10-14h, in theory, it can be administered once on day and the daily dose is 0.6-1.8mg. On April 23 2009, Novo Nordisk announced that Committee for Medicinal products for Human Use (CHMP) under the EMEA gave a positive evaluation on Liraglutide and recommended approval of its listing. Novo Nordisk hopes that European Commission would approve its application of listing within two months.

**BRIEF SUMMARY OF THE INVENTION**

The present invention intends to provide a series of derivatives of GLP-1 analogue which is much more active and has a longer half-life *in vivo.* The derivative of the GLP-1 analogue provide in this invention has the same function as that of human GLP-1 and has a longer half-life *in vivo* compared with human GLP-1.

The present invention also intends to provide a pharmaceutical composition comprising the said derivative of the GLP-1 analogue or a pharmaceutically acceptable salt thereof, for use in the treatment of non-insulin-dependent diabetes mellitus, insulin-dependent diabetes and obesity.

The aims of the present invention are achieved by the following technical solutions. The present invention provides a series of derivatives of GLP-1 analog or a pharmaceutically acceptable salt thereof, wherein the amino acid sequence of said GLP-1 analog is selected from:

His-(D)-Ala-Glu-Gly-Thr-Phe-Thr-Ser-Asp-Leu-Ser-Lys-Gln-Nle-Glu-Glu-Glu-Ala-Val-Arg-Leu-Phe-Ile-Glu-Trp-Leu-Lys-Gln-Gly-Gly-Pro-Ser-Ser-Gly-Ala-Pro-Pro-Pro-Ser-Lys;

His-(D)-Ala-Glu-Gly-Thr-Phe-Thr-Ser-Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser-Lys (SEQ ID No.3);

His-Aib-Glu-Gly-Thr-Phe-Thr-Ser-Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser-Lys (SEQ ID No.4);

His-Ala-Glu-Gly-Thr-Phe-Thr-Ser-Asp-Val-Ser-Ser-Tyr-Leu-Glu-Gly-Gln-Ala-Ala-Glu-Phe-Ile-Val-Trp-Leu-Val-Lys-Gly-Arg-Gly-Lys-Lys-Gly-Gly-Pro-Pro-Pro-Ser-Lys (SEQ ID No.5);

His-Ala-Glu-Gly-Thr-Phe-Thr-Ser-Asp-Val-Ser-Ser-Tyr-Leu-Glu-Gly-Gln-Ala-Ala-Glu-Phe-Ile-Val-Trp-Leu-Val-Lys-Gly-Arg-Gly-Lys-Lys-Gly-Sar-Pro-Pro-Pro-Ser-Lys (SEQ ID No.6);

His-Ala-Glu-Gly-Thr-Phe-Thr-Ser-Asp-Val-Ser-Ser-Tyr-Leu-Glu-Glu-Gln-Ala-Ala-Glu-Phe-Ile-Val-Trp-Leu-Val-Lys-Gly-Arg-Gly-Lys-Lys-Gly-Gly-Pro-Pro-Pro-Ser-Lys (SEQ ID No.7); and

His-Ala-Glu-Gly-Thr-Phe-Thr-Ser-Asp-Val-Ser-Ser-Tyr-Leu-Glu-Glu-Gln-Ala-Ala-Glu-Phe-Ile-Val-Trp-Leu-Val-Lys-Gly-Arg-Gly-Lys-Lys-Gly-Sar-Pro-Pro-Pro-Ser-Lys (SEQ ID No.8),

wherein the derivatives of said GLP-1 analog contains a lipophilic substituent of formula $R_1(CH_2)_n$-CO-, in which $R_1$ is selected from $CH_3$- and HOOC-, n is an integer selected from 8-25, wherein the lipophilic substituent of formula $R_1(CH_2)_n$-CO- and the $\alpha$ amino group of the C-terminal Lys of the GLP-1 analog are linked by an amide bond.

The present disclosure provides a series of derivatives of GLP-1 analogue having amino acid sequence of formula (I) or a pharmaceutically acceptable salt thereof:

X1-X2-Glu-Gly-Thr-Phe-Thr-Ser-Asp-X10-Ser-X12-X13-X14-Glu-X16-X17-Ala-X19-X20-X21-Phe-Ile-X24-Trp-Leu-X27-X28-X29-X30-X31-X32-X33-X34-X35-X36-X37-X38-X39-Lys

(I)

wherein the said derivatives of the GLP-1 analog contain a lipophilic substituent of formula R1(CH2)n-CO-, in which R1 is selected from CH3- and HOOC-, n is an integer selected from 8-25, X1, X2, X10, X12, X13, X14, X16, X17, X19, X20, X21, X24, X27, X28, X29, X30, X31, X32, X33, X34, X35, X36, X37, X38 and X39 are independently selected from any natural or non-natural amino acid or the peptide segments consisting of any natural or non natural amino acid.

The said derivatives of GLP-1 analog refer to a new GLP-1 peptide obtained by the partial amino acid substitution or the extension at the C terminal of human GLP-1 (7-37) peptide serving as a precursor, comprising GLP-1 (7-36) amide and GLP-1 (7-37), which has same function as that of human GLP-1.

The said derivatives refer to make chemical modification to amino acid residues of GLP-1 analog by using lipophilic substituents, wherein a typical modification is to form an amide or ester, preferably, to form an amide.

In a preferred embodiment of the invention, the lipophilic substituent of formula R1(CH2)n-CO- and the amino group of the amino acid residues of the GLP-1 analog are linked by an amide bond, in which R1 is selected from CH3- and HOOC-, and n is an integer selected from 8-25.

In another preferred embodiment of the invention, the lipophilic substituent of formula R1(CH2)n-CO- and the ε amino group of the Lys at the C-terminal of the GLP-1 analog are linked by an amide bond, in which R1 is selected from CH3- and HOOC-, and n is an integer selected from 8-25.

In yet another preferred embodiment of the invention, the lipophilic substituent of formula R1(CH2)n-CO- and the α amino group of the Lys at the C-terminal of the GLP-1 analog are linked by an amide bond, in which R1 is selected from CH3- and HOOC-, and n is an integer selected from 8-25,and 14 is the most preferred.

In another preferred embodiment of the invention, X1 in the amino acid sequence of the GLP-1 analog is selected from L-His and D-His; X2 is selected from Ala, D-Ala, Gly, Val, Leu, Ile, Lys and Aib; X10 is selected from Val and Leu; X12 is selected from Ser, Lys and Arg; X13 is selected from Tyr and Gln; X14 is selected from Leu and Met; X16 is selected from Gly, Glu and Aib; X17 is selected from Gln, Glu, Lys and Arg; X19 is selected from Ala and Val; X20 is selected from Lys, Glu and Arg; X21 is selected from Glu and Leu; X24 is selected from Val and Lys; X27 is selected from Val and Lys; X28 is selected from Lys, Glu, Asn and Arg; X29 is selected from Gly and Aib; X30 is selected from Arg, Gly and Lys; X31 is selected from Gly, Ala, Glu, Pro and Lys; X32 is selected from Lys and Ser; X33 is selected from Lys and Ser; X34 is selected from Gly, Ala and Sar; X35 is selected from Gly, Ala and Sar; X36 is selected from Pro and Gly; X37 is selected from Pro and Gly; X38 is selected from Pro and Gly; X39 is selected from Ser and Tyr.

In one more preferred embodiment of the present invention, the amino acid sequence of the GLP-1 analog is selected from the group consisting of SEQ ID NO: 1 to SEQ ID NO: 120.

In another preferred embodiment of the present invention, the lipophilic substituent of formula R1 (CH2)n-CO- and the amino group of the amino acid residues of the GLP-1 analog, of which the sequence is selected from the group consisting of SEQ ID NO: 1 to SEQ ID NO: 120, are linked by an amide bond, in which R1 is selected from CH3- and HOOC-, and n is an integer selected from 8-25.

In one more preferred embodiment of the present invention, the lipophilic substituent of formula R1 (CH2)n-CO- and the ε amino group of the C-terminal Lys of the GLP-1 analog, selected from the group consisting of SEQ ID NO: 1 to SEQ ID NO: 120, are linked by an amide bond, in which R1 is selected from CH3- and HOOC-, and n is an integer selected from 8-25.

In one more preferred embodiment of the present invention, the lipophilic substituent of formula R1 (CH2)n-CO- and the α amino group of the C-terminal Lys of the GLP-1 analog, selected from the group consisting of SEQ ID NO: 1 to SEQ ID NO: 120, are linked by an amide bond, in which R1 is selected from CH3 and HOOC-, and n is an integer selected from 8-25, preferably n is selected from 8, 10, 12, 14, 16, 18, 20 and 22, most preferably, n is 14.

In one more preferred embodiment of this invention, the lipophilic substituent of formula R1(CH2)n-CO- and the α amino group of the C-terminal Lys of the GLP-1 analog, selected from the group consisting of SEQ ID NO: 1 to SEQ ID NO: 20, are linked by an amide bond, in which R1 is selected from CH3- and HOOC-, and n is an integer selected from 8-25, preferably n is selected from 8, 10, 12, 14, 16, 18, 20 and 22, most preferably, n is 14.

In another more preferred embodiment of this invention, the lipophilic substituent of formula R1 (CH2)n-CO- and the α amido of the C-terminal Lys of the GLP-1 analog, selected from the group consisting of SEQ ID NO: 1 to SEQ ID NO: 8, are linked by an amide bond, in which R1 is CH3, and n is 14.

The derivatives of GLP-1 analogues provided in this invention belong to amphoteric compounds and the person skilled in the art can turn them into salts by using acid or alkaline compounds with known technologies, wherein acids usually used for the formation of acid addition salts are: hydrochloric acid, hydrobromic acid, hydriodic acid, sulfuric acid, phosphoric acid, p-toluenesulfonic acid, methanesulfonic acid, oxalic acid, p-bromophenyl sulfonic acid, carbonic acid, succinic acid, citric acid, benzoic acid, acetic acid; the salts comprise sulfate, pyrosulfate, trifluoroacetate, sulfite, bisulfite, phosphate, biphosphate, dihydric phosphate, metaphosphate, pyrophosphate, hydrochloride, bromide, iodide, acetate, propionate, octanoates, acrylate, formate, isobutyric acid, hexanoate, enanthates, propiolate, oxalate, malonate, succinate, suberate, fumarate, maleate, 1,4 - butynedioate, 1,6-hexynedioate, benzoate, chloro-benzoate, methyl-benzoate, dinitro-benzoate, hydroxyl-benzoate, methoxy-benzoate, phenylacetate, phenpropionate, phenylbutyrate, citrate, lactate, γ-hydroxybutyrate, glycolate, tartrate, methanesulfonate, propanesulfonate, 1-naphthol-sulfonate, 2-naphthol- sulfonate, mandelate and the like, preferably trifluoro-acetate. Alkaline substances can also be turned into salts with derivatives of GLP-1 analogues, wherein the alkaline substances comprise ammonium, hydroxides of alkali metals or alkaline earth metal, and carbonate, bicarbonate, typically sodium hydroxide, potassium hydroxide, ammonium hydroxide, sodium carbonate, potassium carbonate and the like.

The pharmaceutical compositions containing GLP-1 derivatives according to the invention can be used to treat patients who need this treatment by the way of parenteral administration. Parenteral administration can be chosed from subcutaneous, intramuscular or intravenous injections. The GLP-1 derivatives of the invention can also be administered by transdermal routes, such as administration via patch (preferably iontophoresis patch) and administration through the mucosa.

The pharmaceutical compositions containing the GLP-1 derivatives of the invention can be prepared through common techniques in the art of pharmaceutical industry. These techniques comprise proper dissolving and mixing the components to obtain the desired final compositions. For instance, the GLP-1 derivatives are dissolved in a certain amount of water, wherein the volume of water is slightly less than the final volume of the obtained composition. Isotonic agents, preservatives, surfactants and buffers are added according to need, wherein said isotonic agents are sodium chloride, mannitol, glycerol, propylene glycol, sugar or alditol. Said preservatives are phenol, orthocresol, para-cresol, meta-cresol, methylparahydroxybenzoate ester, benzyl alcohol. Said appropriate buffering agents are sodium acetate, sodium carbonate, glycine, histidine, lysine, sodium dihydrogen phosphate, disodium hydrogen phosphate, sodium phosphate. Said surfactants are Poloxamer, Poloxamer -188, Poloxamer-407, Tween 80 and Tween-20. If necessary, the aqueous solutions of acids such as hydrochloric acid or alkali such as sodium hydroxide solution are added to adjust pH values of the solutions, and finally the solution volume is adjusted by adding water to obtain the required concentration. Besides said components, the pharmaceutical compositions of the invention also comprise enough basic amino acids or other alkaline reagents having the same function to decrease the aggregates formed by the composition during storage, such as lysine, histidine, arginine, imidazole during storage.

The derivatives of the GLP-1 analogues of the invention are synthesized manually, wherein the resin is HMPA-AM resin, the α-amino group of the amino acid derivatives is protected by the Fmoc (fluorene formyl carbonyl), the side-chain thiol of cysteine, the side-chain amido of glutamine, the side-chain imidazole of histidine are protected by Trt (triphenylmethyl), the side-chain guanidyl of arginine is protected by Pbf (2,2,4,6,7-pentamethyl-dihydrobenzofuran -5-sulfonyl), the side-chain indolyl of tryptophan and the side-chain amino group of lysine are protected by Boc (tert-Butoxycarbonyl), the side-chain hydroxyl of threonine, the side-chain phenylol of tyrosine, the side-chain hydroxyl of serine are protected by tBu (tert-butyl). The carboxyl of C-terminal amino acids of the single peptide chain of the erythropoietin mimetic peptide derivatives which will be synthesized is connected with an insoluble high molecular resin (rink amind resin) through covalent bonds, and then, the amino acids bound to a solid phase carrier act as amino components, the amino protection group is removed by 20% Hexahydropyridine / DMF solution, and then reactes with excess amino acid derivatives to link a long peptide chain. The operation (Condensation → washing →deprotection→ washing → next round of condensation) is repeated to achieve the peptide chain length desired. Finally, the peptide chain is cleaved down from the resins by using mixture of TFA: water: 1,2-dithioglycol: triisopropylsilane ( 92.5:2.5:2.5:2.5), to obtain the crude derivatives of GLP-1 analogues through precipitation in an ether. The crude peptide monomers are purified through using C18 reversed-phase column, and thereby obtaining the desired derivatives of GLP-1 analogues. The ninhydrin testing method was used to moniter the condensation and the deprotection steps. That is, when there are free aminos on the resin-peptide chain, the ninhydrin reagent will show blue and no color will be shown when there are no free aminos (Ninhydrin reagent itself is yellow). Therefore, after the condensation reaction is completed, if it shows yellow through ninhydrin test (color of Ninhydrin reagent *per se*), then it suggests that the coupling step is completed and the deprotection operation before next amino acid coupling can be carried out; If the testing shows blue, it suggests that there are still some free aminos on the peptide chains, and it is needed to further repeat the coupling step or to change the existing condensing agent till the peptide resins show yellow through ninhydrin test.

**DETAILED DESCRIPTION OF THE INVENTION**

To describe the present invention in more detail, the following examples are provided. However, the present invention should not be construed as limited to the embodiments set forth herein.

Example 1 The method for solid-phase synthesis of HS-20001

1. Preparation of Fmoc-Lys (Mtt)-HMP-AM Resin

(1) drying and swelling of HMP-AM resin

50g (30mmol) HMP-AM resin (0.6mmol/g) dried for 24h in vacuum are placed into a 2L bubblling bottle, resins are swelled with 500mL N, N-dimethylformamide (DMF) for 30 minutes, the DMF is drawn-off, the resins are washed with DMF for 1min, the washing step is repeated twice.

(2)Preparation of Fmoc-Lys (Mtt)-HMP-AM Resin

①Coupling of Fmoc-Lys (Mtt)-OH with HMP-AM resin

The resins are washed with 500mL DCM, and then the washing step is repeated twice. 56.2g (90mmol) Fmoc-Lys (Mtt)-OH and 11.4g (90mmol) DIC are dissolved in 1L DCM, then are added into the swelled HMP-AM resin, then 366mg (3mmol) DMAP are added to react for 24h;

②Washing of the resin

After the reaction, the resin is washed alternately with DMF and IPA twice, washed with DMF for 3 times;

③Capping of hydroxyl

15.3g (150mmol) acetic anhydride and 19.4g (150mmol) DIEA are dissolved in 1L DMF and are added into the resin to react for 10min.

④washing of the resin

The resin is washed twice with 1 L 50% MeOH/DMF, 50% DCM/DMF, and then washed with DCM for three times and with dehydrated ethanol for three times in turn. After dried under vacuum, the Fmoc-Lys (Mtt)-HMP-AM resin is obtained.

(3) Loading assays of Fmoc-Lys(Mtt)-HMP-AM resin

Accurate 5 ~ 10 mg resin are put into 1mL 20% Hexahydropyridine / DMF solution, stirred for 20min, then 50uL supernatant is taken with a pipet and is diluted in 2.5ml DMF;

Blank samples: 50uL 20% Hexahydropyridine/DMF is taken with a pipet and is diluted in 2.5ml DMF;
Degree of substitution is calculated as follows:

$$Sub=(A\times51)/(7.8\times m)$$

wherein A is the absorption value of UV at 301nm; m is the weight of the resin, the unit is mg.

2. Drying and swelling of the solid-phase synthesized resin

50g (20mmol) Fmoc-Lys (Mtt)-HMPA-AM resin (0.4mmol/g) dried in vacuum for 24h are placed into a 2L bubbling bottle, then 500ml N, N-dimethylformamide (DMF) are added to swell the resin for 30min, then the DMF solution is drawn-off.

3. Removal of 4 - methyl triphenylmethyl (Mtt) protecting group of Fmoc-Lys (Mtt)-HMPA-AM resin

The resin is washed with 200ml DCM twice, then 1200mL 1% TFA / DCM (TFA is about 8-fold excess) are added to remove Mtt protecting group for 1h, the resin is alternately washed with 200mL 5% N, N-diisopropyl ethylamine (DIEA) / DMF and DMF for three times, then washed with DMF for 3 times;

4. Palmitic acid condensation

50mmol palmitic acid and 50mmol 3 - (diethoxyphosphoryloxy) -1,2,3 - phentriazine -4- ketone (DEPBT) are dissolved in 400ml DMF, then 100mmol DIEA are added and are stirred for 3min at room temperature, said solution is added to the resin, reacted in 37°C water baths for 2h under $N_2$. After the reaction, the reaction solution is drawn-off, the resin is washed with DMF, isopropyl alcohol (IPA) and DMF in turn.

5. Removal of 9- Fmoc (fluorenylmethyloxycarbonyl) protecting group of Fmoc-Lys (N-ε-palmitic acid)-HMPA-AM resin

200mL 20% piperidine/DMF solution are placed into a bubbling bottle filled with Fmoc-Lys (Mtt)-HMPA-AM resin, reacted for 5min and then is drawn out, then 200mL 20% piperidine/DMF solution are added to react for 20min at room temperature. After the reaction, the resin is washed with 200mL DMF for four times.

6. Solid phase synthesis of peptide chain part of HS-20001

① Condensation of Fmoc-Ser (tBu)-OH

50mmol Fmoc-Ser (tBu)-OH are dissolved in 125mL 0.4M 1-hydroxybenzo triazole (HOBt) / DMF, then 125ml 0.4M N, N'-diisopropyl carbodiimide (DIC) / DCM are added to activate and react for 10min at room temperature; said solution is added into the resin, reacted under protection of nitrogen at room temperature, and ninhydrin is used to detect and control the degree of the reaction. After the reaction, the reaction solution is removed; the resin is washed with DMF, IPA and DMF in turn.

① Extension of the peptide chain

HS-20001resin peptide is synthesized according to the sequence of the peptide chain of HS-20001 from the amino terminal (N-terminal) to the carboxy-terminal (C-terminal) (His-(D)-Ala-Glu-Gly-Thr-Phe-Thr-Ser- Asp-Leu-Ser-Lys-Gln-Nle-Glu-Glu-Glu-Ala-Val-Arg-Leu-Phe-Ile-Glu-Trp-Leu-Lys-Gln-Gly-Gly-Pro-Ser-Ser-Gly-Ala-Pro-Pro-Pro-Ser), wherein the amounts of amino acids and condensation reagents are the same as the amounts for Fmoc-Ser (tBu)-OH, protected amino acids are Fmoc-Ser(tBu)-OH, Fmoc-Pro-OH, Fmoc-Ala-OH, Fmoc-Gly-OH, Fmoc-Gln(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Arg(Pbf)-OH, Fmoc-Val-OH, Fmoc-Nle-OH, Fmoc-Asp(OtBu)-OH, Fmoc-Thr(tBu)-OH, Fmoc-D-Ala-OH and Fmoc-His(Trt)-OH respectively, and condensation and deprotection reactions are repeated.

③ Post-processing of HS-20001 resin peptide

Said HS-20001 resin peptide obtained in step ② is washed with DMF, IPA and DMF in turn, washed with absolute ether twice, then dried under vacuum, and the HS-20001 resin peptide is obtained.

④ Preparation of HS-20001 crude peptide

The dried HS-20001 peptide resins reacted with fresh lysate), of trifluoroacetic acid (TFA): triisopropylsilane (TIS): water = 95:2.5:2.5 (by volume and total 10mL of lysate per gram of the dry resin) for 4h at room temperature . The reaction solution is filtrated after the reaction, the resin is washed with TFA twice, the filtrate is collected and combined, and is concentrated to 1/3 of the original volume through rotary evaporation, HS-20001 is precipitated and washed with cold absolute ether, after centrifugation and drying in vacuum, white crude HS-20001 is obtained.

⑤Preparation of HS-20001 with reversed-phase liquid chromatography

10g crude HS-20001 are dissolved in a certain amount of water, filtrated with 0.45$\mu$m membrane filter, then purified with reversed-phase high performance liquid chromatography (RP-HPLC), wherein the mobile phase is A 0.1% TFA/$H_2$O, B 0.1% TFA / acetonitrile,
wherein, the column is Denali C-18 column (particle diameter 8.3$\mu$m, 5$\times$30cm), column temperature is 45°C, detection wavelength is 220nm, flow rate is 120mL/min. The product peaks are collected, concentrated under the vacuum to remove most of acetonitrile, then the product of HS-20001 2.25g are obtained by lyophilization, of which the purity is 98.5%, the yield is 22.5%.

Example 2 The solid-phase synthesis method for HS-20002

1. Preparation of Fmoc-Lys (Mtt)-HMP-AM Resin

See Example 1.

2. Drying and swelling of the solid-phase synthesized resin

50g (20mmol) Fmoc-Lys(Mtt)-HMPA-AM resin (0.4mmol/g) dried for 24 in vacuum are placed into a 2L bubbling bottle, the resin is swelled with 500mL DMF for 30min, then DMF solution is drawn-off.

3. Removal of Mtt protecting group of Fmoc-Lys (Mtt)-HMPA-AM resin

The resin is washed with 200mL DCM twice, Mtt protecting group is removed by adding 1200mL 1% TFA / DCM (TFA is about 8-fold excess) for 1h, then washed with 200mL 5% DIEA/DMF and DMF alternately for three times, then washed with DCM for three times.

4. Palmitic acid Condensation

50mmol palmitic acid and 50mmol DEPBT are dissolved in 400mLDMF, and then 100mmol DIEA are added by stirring to react for 3min at room temperature, the said solution is added to the resin, reacted in 37°C water baths under N2 for 2h. After the reaction, the reaction solution is removed and the resin is washed with DMF, isopropyl alcohol (IPA) and DMF in turn.

5. Removal of Fmoc protecting group of Fmoc-Lys (N-$\varepsilon$-palmitic acid)-HMPA-AM resin

200ml 20% Piperidine / DMF solution are placed into a bubbling bottle filled with Fmoc-Lys(Mtt)-HMPA-AM resin, drawn-off after reacting for 5min, then 200ml 20% Piperidine / DMF solution are added for reacting for 20min at room temperature. After the completion of the reaction, the resin is washed for four times with 200ml DMF.

6. Solid-phase synthesis method for the peptide chain part of HS-20002

① Condensation of Fmoc-Ser (tBu)-OH

50mmol Fmoc-Ser(tBu)-OH are dissolved in 125mL 0.4M HOBt/DMF, then 125mL 0.4M DIC/DCM are added to activate and react for 10min at room temperature; the said solution is placed into the resin and reacted under $N_2$ at room temperature, and ninhydrin testing is used to detect and control the degree of the reaction. After the reaction, the reaction solution is removed, and the resin is washed with DMF, IPA and DMF in turn.

①Extension of the peptide chain

HS-20002 resin peptide is synthesized according to the sequence of peptide chain of HS-20002 from the N-amino (N-terminal) to the carboxy-terminal (C-terminal) (His-Gly-Glu-Gly-Thr-Phe-Thr-Ser-Asp -Leu-Ser-Lys-Gln- Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser), wherein the amounts of amino acids and condensation reagents are as same as that of Fmoc-Ser (tBu)-OH, protected amino acids are Fmoc-Ser(tBu)-OH, Fmoc-Pro-OH, Fmoc-Ala-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Arg(Pbf)-OH, Fmoc-Val-OH, Fmoc-Met-OH, Fmoc-Gln(Trt)-OH, Fmoc-Asp(OtBu)-OH, Fmoc-Thr(tBu)-OH and Fmoc-His(Trt)-OH respectively, and condensation and deprotection reactions are repeated.

③ Post-processing of HS-20002 resin peptide

Said HS-20002 resin peptide obtained in step ② is washed with DMF, IPA and DMF in turn, washed twice with absolute ether, then dried under the vacuum, HS-20002 resin peptide is obtained ultimately.

④ Preparation of HS-20002 crude peptide

The dried HS-20002 peptide resin is reacted with fresh lysate of trifluoroacetic acid (TFA): triisopropylsilane (TIS): water: 1,2-ethanedithiol (EDT)= 94:1:2.5:2.5 (by volume and total 10mL of lysate per gram of the dry resin)for 4h at room temperature . The reaction solution is filtrated after the reaction, the resin is washed with TFA twice, the filtrate is collected and combined, and is concentrated to 1/3 of the original volume through rotary evaporation, HS-20002 is precipitated with cold absolute ether, after centrifugation and drying under vacuum, white crude HS-20002 is obtained.

⑤ Preparation of HS-20002 with reversed-phase liquid chromatography

10g crude HS-20002 are dissolved in a certain amount of water, filtrated with $0.45\mu m$ membrane filter, then purified with reversed-phase high performance liquid chromatography (RP-HPLC), wherein mobile phase is A 0.1% TFA/$H_2O$, B 0.1% TFA / acetonitrile, wherein the column is Denali C-18 column (particle diameter $8.3\mu m$, $5\times30cm$), column temperature is 45°C, detection wavelength is 220nm, flow rate is 120mL/min. The product peaks are collected, concentrated under vacuum to remove most of acetonitrile, then the product of HS-20002 2.1g are obtained by lyophilization, of which the purity is 98%, the yield is 20.5%.

Example 3 The solid-phase synthesis method for HS-20003

1. Preparation of Fmoc-Lys (Mtt)-HMP-AM Resin

See Example 1.

2. Drying and swelling of the solid-phase synthesized resin

50g (20mmol) Fmoc-Lys (Mtt)-HMPA-AM resin (0.4mmol/g) dried for 24h in vacuum are placed into a 2L bubbling bottle, the resin is swelled with 500mL DMF for 30min, then DMF solution is drawn-off.

3. Removal of Fmoc protecting group of Fmoc-Lys (Mtt)-HMPA-AM resin

200mL 20% piperidine/DMF solution are added into a bubbling bottle filled with Fmoc-Lys (Mtt)-HMPA-AM resin, the solution is drawn off after 5min, and then 200mL 20% piperidine/DMF solution are added and let react for another 20min at room temperature. After the reaction, the resin is washed with 200mL DMF for four times.

4. Palmitic Acid Condensation

50mmol Palmitic acid and 50mmol DEPBT are dissolved in 400mLDMF, then 100mmol DIEA are added by stirring to react for 3min at room temperature, the said solution is added to the resin, reacted in 37°C water baths under $N_2$ for 2h. After the reaction, the reaction solution is removed and the resin is washed with DMF, isopropyl alcohol (IPA) and DMF in turn.

5. Removal of Mtt protecting group of Palmitic acid-Lys(Mtt)-HMPA-AM resin

The resin is washed with 200mL DCM twice, Mtt protecting group is removed by adding 1200mL 1% TFA / DCM (TFA is about 8-fold excess) to react for 1h, The resin is washed with 200mL 5% DIEA/DMF and DMF alternately for three times, then washed with DCM for three times.

6. Solid-phase synthesis method for the peptide chain part of HS-20003

① Condensation of Fmoc-Ser (tBu)-OH

50mmol Fmoc-Ser (tBu)-OH and 50mmol DEPBT are dissolved in a certain amount of DCM, then 100mmol DIEA are added for activation for 3min at room temperature; the said solution is added to the resin, reacted under $N_2$ at room temperature, and ninhydrin is used to detect and control the degree of the reaction. After the reaction, the reaction solution is removed and the resin is washed with DMF, IPA and DMF in turn.

①Extension of the peptide chain

HS-20003 resin peptide is synthesized according to the sequence of peptide chain of HS-20003 from the N-amino (N-terminal) to the carboxy-terminal (C-terminal) (His-(D)-Ala-Glu-Gly-Thr-Phe-Thr-Ser -Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser), wherein the amounts of amino acids and condensation reagents are as same as that of Fmoc-Ser (tBu)-OH, protected amino acids are Fmoc-Ser(tBu)-OH, Fmoc-Pro-OH, Fmoc-Ala-OH, Fmoc-Gly-OH, Fmoc-Arg(Pbf)-OH, Fmoc-Val-OH, Fmoc-Leu-OH, Fmoc-Trp(Boc)-OH, Fmoc-Ile-OH, Fmoc-Phe-OH, Fmoc-Glu(OtBu)-OH, Fmoc-Lys(Boc)-OH, Fmoc-Tyr(tBu)-OH, Fmoc-Asp(OtBu)-OH, Fmoc-Thr(tBu)-OH respectively, and condensation and deprotection reactions are repeated.

③ Post-processing of HS-20003 resin peptide

Said HS-20003 resin peptide obtained in step ② is washed with DMF, IPA and DMF in turn, washed twice with absolute ether, then dried under the vacuum and HS-20003 resin peptide is obtained ultimately.

④ Preparation of HS-20003 crude peptide

The dried HS-20003 peptide resin is reacted with fresh lysate of trifluoroacetic acid (TFA): triisopropylsilane (TIS): water = 95:2.5:2.5 (by volume and total 10mL of lysate per gram of the dry resin)for 4h at room temperature . The reaction solution is filtrated after the reaction, the resin is washed with TFA twice, the filtrate is collected and combined, and is concentrated to 1/3 of the original volume through rotary evaporation, HS-20003 is precipitated with cold ether under stirring, after centrifugation and drying in vacuum, white crude HS-20003 is obtained ultimately.

⑤ Preparation of HS-20003 with reversed-phase liquid chromatography

10g crude HS-20003 are dissolved in a certain amount of 20% acetic acid / water and stirred for at least 4h, then filtrated with 0.45$\mu$m membrane filter, then purified with reversed-phase high performance liquid chromatography (RP-HPLC), wherein mobile phase is A 0.1% TFA/$H_2O$, B 0.1% TFA / acetonitrile, wherein, the column is Denali C-18 column (particle diameter 8.3$\mu$m, 5×30cm), column temperature is 45°C, detection wavelength is 220nm, flow rate is 120mL/min. The product peaks are collected, concentrated with decompression to remove most of acetonitrile, then the product of HS-20003 2.5g are obtained by lyophilization, of which the purity is 98.5%, the yield is 25%.

Example 4 The solid-phase synthesis method for HS-20004

1. Preparation of Fmoc-Lys (Mtt)-HMP-AM Resin

See Example 1.

2. Drying and swelling of the solid-phase synthesized resin

50g (20mmol) Fmoc-Lys(Mtt)-HMPA-AM resin (0.4mmol/g) dried for 24 under the vacuum are placed into a 2L bubbling bottle, 500mL DMF are added to swell the resin for 30min, DMF solution is drawn-off.

3. Removal of Fmoc protecting group of Fmoc-Lys (Mtt)-HMPA-AM resin

200mL 20% piperidine/DMF solution are added into a bubbling bottle filled with Fmoc-Lys (Mtt)-HMPA-AM resin, and then drawn off after 5min, and then 200mL 20% piperidine/DMF solution are added for reacting for 20min at room temperature. After the reaction, the resin is washed with 200mL DMF for four times.

4. Palmitic acid Condensation

50mmol palmitic acid and 50mmol DEPBT are dissolved in 400mLDMF, and then 100mmol DIEA are added by stirring for 3min at room temperature, the said solution is added to the resin, reacted in 37°C water baths under $N_2$ for 2h. After the reaction, the reaction solution is removed and the resin is washed with DMF, isopropyl alcohol (IPA) and DMF in turn.

5. Removal of Mtt protecting group of Palmitic acid-Lys(Mtt)-HMPA-AM resin

The resin is washed with 200mL DCM twice, Mtt protecting group is removed by adding 1200mL 1% TFA / DCM (TFA is about 8-fold excess) for reacting for 1h, then washed with 5% DIEA/DMF and DMF alternately for three times, then washed with DCM for three times.

6. The solid-phase synthesis method for the peptide chain part of HS-20004

① Condensation of Fmoc-Ser (tBu)-OH

50mmol Fmoc-Ser(tBu)-OH and 50mmol DEPBT are dissolved in a certain amount of DCM, then 100mmol DIEA are added for activation for 3min at room temperature; the said solution is added to the resin, reacted under $N_2$ at room temperature, and ninhydrin is used to detect and control the degrees of the reaction. After the reaction, the reaction solution is removed; the resin is washed with DMF, IPA and DMF in turn.

② Extension of the peptide chain

HS-20004 resin peptide is synthesized according to the sequence of the peptide chain of HS-20004 from the N-amino (N-terminal) to the carboxy-terminal (C-terminal) (His-Aib-Glu-Gly-Thr-Phe-Thr-Ser -Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser), wherein the amounts of amino acids and condensation reagents are as same as that of Fmoc-Ser (tBu)-OH, protected amino acids are Fmoc-Ser(tBu)-OH, Fmoc-Pro-OH, Fmoc-Ala-OH, Fmoc-Gly-OH, Fmoc-Arg(Pbf)-OH, Fmoc-Val-OH, Fmoc-Leu-OH, Fmoc-Trp(Boc)-OH, Fmoc-Ile-OH, Fmoc-Phe-OH, Fmoc-Glu(OtBu)-OH, Fmoc-Lys(Boc)-OH, Fmoc-Tyr(tBu)-OH,Fmoc-Asp(OtBu)-OH,Fmoc-Thr(tBu)-OH,Fmoc-Aib-OH and Fmoc-His(Trt)-OH respectively and the condensation and deprotection reactions are repeated.

③ Post-processing for HS-20004 resin peptide

Said HS-20004 resin peptide obtained in step ② is washed with DMF, IPA and DMF in turn, washed twice with absolute ether, then dried under the vacuum, HS-20004 resin peptide is finally obtained.

④ Preparation of crude HS-20004 peptide

The dried HS-20004 resin peptide is reacted with fresh lysate of trifluoroacetic acid (TFA): triisopropylsilane (TIS): water = 95:2.5:2.5 (by volume and total 10mL of lysate per gram of the dry resin)for 4h at room temperature. The reaction solution is filtrated after the reaction, the resin is washed with TFA twice, the filtrate is collected and combined , and is concentrated to 1/3 of the original volume through rotary evaporation, HS-20004 is precipitated with cold ether under stirring , after centrifugation and drying in vacuum, white crude HS-20004 is obtained ultimately.

⑤ Preparation of HS-20004 with reversed-phase liquid chromatography

10g crude HS-20002 are dissolved in a certain amount of 20% acetic acid / water and stirred for at least 4h, then filtrated with 0.45$\mu$m membrane filter, then purified with reversed-phase high performance liquid chromatography (RP-HPLC), wherein mobile phase is A 0.1% TFA/$H_2O$, B 0.1% TFA / acetonitrile, the gradient is as follows: wherein, the column is Denali C-18 column (particle diameter 8.3$\mu$m, 5×30cm), column temperature is 45°C, detection wavelength is 220nm, flow rate is 120mL/min. The product peaks are collected, concentrated with under the vacuum to remove most of acetonitrile, then the product of HS-20004 2.25g are obtained by lyophilization, of which the purity is 98.5%, the yield is 22.5%.

Example 5 The solid-phase synthesis method for HS-20005

The preparation method of HS-20005 is as same as that described in example 4, wherein the difference is that the amino acid sequence is replaced with SEQ ID NO: 5, and 2.5g HS-20005 product is obtained, of which the purity is 98.5%, the yield is 25%.

Example 6 The solid-phase synthesis method for HS-20006

The preparation method of HS-20006 is as same as that described in example 4, wherein the difference is that the amino acid sequence is replaced with SEQ ID NO: 6, and 2.25g HS-20006 product is obtained, of which the purity is 98.5%, the yield is 22.5%.

Example 7 The solid-phase synthesis method for HS-20007

The preparation method of HS-20007 is as same as that described in example 4, wherein the difference is that the amino acid sequence is replaced with SEQ ID NO: 7, and 2.1g HS-20007 product is obtained, of which the purity is 98%, the yield is 20.5%.

Example 8 The solid-phase synthesis method for HS-20008

The preparation method of HS-20008 is as same as that described in example 4, wherein the difference is that the amino acid sequence is replaced with SEQ ID NO: 8, and 2.5g HS-20008 product is obtained, of which the purity is 98.5%, the yield is 25%.

**Reference example solid-phase synthesis method for Liraglutide**

1. Preparation of Fmoc-Lys(Mtt)-HMP-AM resin

(1) Drying and swelling of HMP-AM resin

50g (30mmol) HMP-AM resin (0.6mmol/g) dried for 24h in vacuum are placed into a 2L bubbling bottle, 500mL N, N-dimethylformamide (DMF) are added to swell the resin for 30min, the DMF solution is drawn-off, DMF is added to wash the resin for 1min, the washing step is repeated twice.

(2) Preparation of Fmoc-Lys(Mtt)-HMP-AM Resin

① Coupling of Fmoc-Lys (Mtt)-OH and HMP-AM resin

The resin is washed with 500mL DCM for three times, 56.2g (90mmol) Fmoc-Lys(Mtt)-OH and 11.4g (90mmol) DIC are dissolved in 1L DCM, then added into the swelled HMP-AM resin, then 366mg(3mmol) DMAP are added to let react for 24h;

② Washing of the resin

After the reaction, the resin is washed alternately with DMF and IPA twice, washed with DMF for 3 times;

③ Capping of hydroxyl

15.3g (150mmol) acetic anhydride and 19.4g (150mmol) DIEA are dissolved in 1L DMF and added to the resin for reacting for 10min.

④ washing of the resin

The resin is washed with 1 L 50% MeOH/DMF, 50% DCM/DMF twice, washed with DCM for three times, washed with absolute ethanol for three times, then dried under vacuum to obtain the Fmoc-Lys(Mtt)-HMP-AM resin.

(3) Loading assays of Fmoc-Lys(Mtt)-HMP-AM resin

Accurate 5 ~ 10 mg resin are put into 1mL 20% Hexahydropyridine / DMF solution, stirred for 20min, then 50uL supernatant is taken with a pipet and is diluted in 2.5ml DMF;
Blank samples: 50uL 20% Hexahydropyridine/DMF is taken with a pipet and is diluted in 2.5ml DMF;
Degree of substitution is calculated as follows:

$$Sub = (A \times 51)/(7.8 \times m)$$

wherein A is the absorption value of UV at 301nm; m is the amount of the resin, the unit is mg.

2. Drying and swelling the resin of the solid-phase synthesis

50g (20mmol) Fmoc-Gly-HMP-AM resin (0.4mmol/g) dried for 24h in vacuum are placed into a 2L bubbling bottle, then 500ml N, N-dimethylformamide (DMF) are added to swell the resin for 30min, then the DMF solution is drawn-off.

3. The solid phase synthesis method of the peptide chain part of Liraglutide

① condensation of Fmoc-Arg(Pbf)-OH

50mmol Fmoc-Arg(Pbf)-OH are dissolved in 125mL 0.4M 1-hydroxybenzotriazole (HOBt) / DMF, then 125ml 0.4M N, N'-diisopropylcarbodiimide (DIC) / DCM are added to activate and react for 10min at room temperature; said solution is added to the resin, reacted under $N_2$ at room temperature, and ninhydrin testing is used to detect and control the degrees of the reaction. After the reaction, the reaction solution is drawn off; the resin is washed with DMF, IPA and DMF in turn.

② Extension of the peptide chain

Precursor peptide of Liraglutide is synthesized according to the sequence of the peptide chain of Liraglutide from the N-amido (N-terminal) to the carboxy-terminal (C-terminal) (His-Ala-Glu-Gly-Thr-Phe-Thr-Ser-Asp- Val-Ser-Ser-Tyr-Leu-Glu-Gly-Gln-Ala-Ala-Lys-Glu-Phe-Ile-Ala-Trp-Leu-Val-Arg-Gly-Arg-Gly), wherein the amounts of amino acids and condensation reagents are the same as that of Fmoc-Arg(Pbf)-OH, protected amino acids are Fmoc-Arg(Pbf)-OH, Fmoc-Val-OH, Fmoc-Leu-OH, Fmoc-Trp(Boc)-OH, Fmoc-Ala-OH, Fmoc-Ile-OH, Fmoc-Phe-OH, Fmoc-Glu(OtBu)-OH, Fmoc-Lys(Mtt)-OH, Fmoc-Gln(Trt)-OH, Fmoc-Tyr(tBu)-OH, Fmoc-Ser(tBu)-OH, Fmoc-Asp(OtBu)-OH, Fmoc-Thr(tBu)-OH, Fmoc-His(Trt)-OH respectively, and the condensation and deprotection reactions are repeated.

③ Removal of Mtt protecting group of the precursor peptide of Liraglutide

The resin is washed with 200ml DCM twice, Mtt protecting group is removed twice by adding 1200mL 1% TFA / DCM (TFA is about 8-fold excess) to react for 1h, the resin is washed alternately with 200mL 5% N, N-diisopropylethylamine (DIEA) / DMF and DMF for three times, washed for 3 times with DMF;

④ Modification of precursor peptide of Liraglutide with palmitic acid

50mmol Fmoc-Glu-OtBu are dissolved in 125mL 0.4M 1-hydroxybenzo triazole (HOBt) / DMF, then 125ml 0.4M N, N'-diisopropylcarbodiimide (DIC) / DCM is added to activate and react for 10min at room temperature; said solution is added to the resin, reacted under $N_2$ at room temperature, and ninhydrin is used to detect and control the degrees of the reaction. After the reaction, the reaction solution is drawn-off, the resin is washed with DMF, IPA and DMF in turn.
1 L 20%PIP/DMF are added to remove Fmoc protecting group for 5min, then are drawn off, then 1 L 20%PIP/DMF are added to remove Fmoc protecting group for 20min, then are drawn off, the resin is washed with DMF for four times;
50mmol palmitic acid and 50mmol 3 - (diethoxyphosphoryloxy) -1,2,3 - phentriazine -4- ketone (DEPBT) are dissolved in 400ml DMF, then 100mmol DIEA are added to react for 3min under stirring at room temperature, said solution is added to the resin, reacted in 37°C water baths under $N_2$ for 2h. After the reaction, the reaction solution is drawn-off, the resin is washed with DMF, isopropyl alcohol (IPA) and DMF in turn.

EP 2 460 825 B1

4. Post-processing of the resin peptide of Liraglutide

Said resin peptide of Liraglutide obtained in step (2) is washed with DMF, IPA and DMF in turn, washed for three times with DCM, washed twice with absolute ether, then dried in vacuum, resin peptide of Liraglutide is obtained ultimately.

5. Preparation of crude peptide of Liraglutide

The dried peptide resin of Liraglutide is reacted with fresh lysate of trifluoroacetic acid (TFA): triisopropylsilane (TIS): water = 95:2.5:2.5 (by volume and total 10mL of lysate per gram of the dry resin) for 4h at room temperature. The reaction solution is filtrated after the reaction, the resin is washed with TFA twice, the filtrate is collected and combined, concentrated to 1/3 of the original volume through rotary evaporation, Liraglutide is precipitated with cold absolute ether, after centrifugation and drying under the vacuum, white crude HS-20001 is obtained.

⑤ Preparation of Liraglutide with reversed-phase liquid chromatography

10g crude Liraglutide are dissolved in a certain amount of $NH_4HCO_3$ solution, filtrated with 0.45$\mu$m membrane filter, then is purified with reverse-phase high performance liquid chromatography (RP-HPLC), wherein mobile phase is A 0.1% TFA/$H_2O$, B 0.1% TFA / acetonitrile, wherein, the column is Denali C-18 column (particle diameter 8.3$\mu$m, 5×30cm), column temperature is 45°C, detection wavelength is 220nm, flow rate is 120mL/min. The product peaks are collected, concentrated with decompression to remove most of acetonitrile, then 2.25g product of Liraglutide is obtained by lyophilization, of which the purity is 98%, the yield is 12.5%.

Experimental Example 1: Testing the agonist activity of the compounds on glucagon-like peptide-1 receptor (GLP1R)

GLP1R is a receptor coupled with Gs protein, of which the binding to the agonists will result in the increasing of intracellular cAMP concentration. In the present experiment, GLP1R and the luciferase reporter gene plasmid regulated by cAMP response elements are co-transfected into HEK293 cells. When the compound binds to the receptor and activates the receptors, the expression of the luciferase will increase. The activation status of of the compound to GLP1R can be learned by testing the activity of the luciferase.

| No. of the test drugs : | amount (mg) | DMSO (ul) | Final concentration (mM) |
|---|---|---|---|
| Liraglutide | 2 | 53.31556 | 10 |
| HS-20001 | 2 | 43.81871 | 10 |
| HS-20002 | 2 | 43.91502 | 10 |
| HS-20003 | 2 | 44.99387 | 10 |
| HS-20004 | 2 | 44.8526 | 10 |
| HS-20005 | 2 | 43.81871 | 10 |
| HS-20006 | 2 | 43.91502 | 10 |
| HS-20007 | 2 | 44.99387 | 10 |
| HS-20008 | 2 | 44.8526 | 10 |

Experimental procedures:

1. HEK293 cells stably transfected with GLP1R and pCRE-Luc plasmid are implanted in 96 well plate with the amount 40000 cells / well / 100 $\mu$l, and incubated at 37°C for 24h.

2. The compounds or positive drugs having a certain concentration gradient are added (3 wells per concentration) and incubated at 37°C for 5h. The negative control is solvent DMSO.

3. 50$\mu$l culture medium are taken from each well and 50$\mu$l the substrate of the luciferase are added and then vortexed

17

for 10min.

4. 80μl reaction solution are taken and transferred to a white 96 well plate, then detected on the Invision microplate reader (enzyme-labelling measuring instrument).

The experimental results: compared with positive compounds liraglutide, the activity of the compound HS-20001 of the invention is approximately equal to that of the positive compounds, but HS-20002-20008 show much better agonist activity.

Table 1. EC50 Values of the series of compounds:

| Compounds | EC50 (nM) | 95% CI (nM) | Maximum reaction rate (%) |
|---|---|---|---|
| Liraglutide | 0.014707 | 9.726e-012 to 2.223e-011 | 96.84616 |
| HS-20001 | 0.013552 | 7.6757e-012 to 2.3963e-011 | 98.11013 |
| HS-20002 | 0.0014145 | 1.2036e-012 to 1.6623e-012 | 87.99447 |
| HS-20003 | 0.00071876 | 4.9657e-013 to 1.0404e-012 | 87.86082 |
| HS-20004 | 0.00037259 | 2.1453e-013 to 6.4710e-013 | 90.81368 |
| HS-20005 | 0.00023552 | 7.3567e-012 to 2.2346e-011 | 89.13468 |
| HS-20006 | 0.00064358 | 1.3581e-012 to 1.4523e-012 | 87.4281 |
| HS-20007 | 0.00054921 | 4.1354e-013 to 1.2514e-012 | 87.0389 |
| HS-20008 | 0.00021002 | 2.2436e-013 to 6.0245e-013 | 88.4628 |

Experimental Example 2 Test of the activity *in vivo*

db/db mice with type 2 diabetes are divided into six groups based on the random blood glucose and body weight (8 per group). Physiological saline, 3 or 10μg/kg HS series new compounds (Liraglutide, 20001, 20002, 20003, 20004, 2005, 2006, 2007, 2008) are administered by single subcutaneous injection. The random blood glucose of the mice is determined at different time after administration.

The animals subjects used in the experiment are db/db mice, which are products of a U.S. corporation named Jackson and are conserved and reproduced by Shanghai Institute of Materia Medica of Chinese Academy of Science, of which the Certificate of Conformity is : SCXK(HU)2008-0017, Body Weight: 35-50g; Gender: Male 85, female 86, bred in SPF-grade animal room; Temperature: 22-24°C; Humidity: 45-80%; Light: 150-300Lx, 12h day alternates with night.

The test drugs of the experiment are HS-20001, HS-20002, HS-20003, HS-20004, HS-20005, HS-20006, HS-20007, HS-20008, liraglutide (developed by Novo Nordisk, as Positive control).

Preparation method: 1 bottle of the compound (2mg/bottle) is dissolved with double-distilled water to prepare a colorless and transparent solution of which the concentration is 2mg/ml, then the solution is diluted to 0.6μg/ml and 2μg/ml with physiological saline (Sodium chloride injection, Double-Crane Pharmaceutical Co., Ltd. Anhui, batch number: 080728 6C). "ACCU-CHEK® Advantage" blood glucose meter from Roche is used to determine the blood glucose.

**Dose setting and group**

**Test Group 1:**

Control group: physiological saline

Liraglutide group 3μg/kg
HS-20001 group :    3μg/kg
HS-20002group :    3μg/kg
HS-20003group :    3μg/kg
HS-20004group :    3μg/kg
HS-20005group :    3μg/kg
HS-20006group :    3μg/kg

(continued)

| | |
|---|---|
| HS-20007 group : | 3μg/kg |
| HS-20008 group : | 3μg/kg |

**Test Group 2:**

| | |
|---|---|
| Control group: physiological saline | |
| Liraglutide group : | 10μg/kg |
| HS-20001 group : | 10μg/kg |
| HS-20002 group : | 10μg/kg |
| HS-20003 group : | 10μg/kg |
| HS-20004 group : | 10μg/kg |
| HS-20005 group : | 10μg/kg |
| HS-20006 group : | 10μg/kg |
| HS-20007 group : | 10μg/kg |
| HS-20008 group : | 10μg/kg |

Route and volume of administration: Single subcutaneous injection dose, dose volume is 5ml/kg.

Test Method

Screening, grouping, and administration for db/db mice with type 2 diabetes

Test Group 1:

171 db/db mice (male 85, female 86) are single-cage reared after weaning, fed with high fat diet. The random and fasting blood glucoses are measured after the db/db mice are seven weeks old. 80 db/db mice which fall ill are picked out and are divided into 10 groups according to random blood glucose, fasting blood glucose and body weight as follows: model control group, Liraglutide group-3μg/kg, HS-20001 group-3μg/kg, HS-20002 group-3μg/kg, HS-20003 group-3μg/kg, HS-20004 group-3μg/kg, HS-20005 group-3μg/kg, HS-20006 group-3μg/kg, HS-20007 group-3μg/kg and HS-20008 group-3μg/kg.

Test Group 2:

The random blood glucoses of db/db mice are measured. 80 db/db mice which fall ill are picked out and are divided into 10 groups according to random blood glucose and body weight as follows: model control group, Liraglutide group-10μg/kg, HS-20001 group-10μg/kg, HS-20002 group-10μg/kg, HS-20003 group-10μg/kg, HS-20004 group-10μg/kg, HS-20005 group-10μg/kg, HS-20006 group-10μg/kg, HS-20007 group-10μg/kg and HS-20008 group-10μg/kg.
Each group has 8 mice, half male and half female. The animals of each group are administered with the test compounds or solvent control respectively through single subcutaneous injection. The random blood glucose is determined at 1h, 2h, 4h, 8h and 24h after administration and the decrease rate of blood glucose is calculated:

$$\text{Decrease rate of blood glucose} = (\text{blood glucose of control group} - \text{blood glucose of treatment group}) / \text{blood glucose of control group} * 100\%.$$

The experimental results

Test 1: Effect of the low-dose new compounds administered by singe dose on random blood glucose of db/db mice

The results can be seen in Table 2 and Table 3. db/db mice are administered with 3μg/kg HS-20002, 20004, 20005, 20006, 20007 or 20008 through single subcutaneous injection, after one hour, random blood glucose values of the said mice are decreased significantly compared with those of the control group (P <0.05), decrease rates are 24.51%, 15.00%, 14.00%, 14.25%, 13.98% and 13.90% respectively; after 2h and 4h from administration, random blood glucose values keep a lower level and have significant difference from those of the control group (P <0.05); after 8h from the administration, random blood glucose values have no significant difference from those of the control group. The mice are administered with 3μg/kg HS-20003 through subcutaneous injection, after one hour, random blood glucose values are decreased significantly compared with those of the control group (P <0.05), up to 17.33%, after 2h, 4h and 8h from the administration, random blood glucose values show no significant difference from those of the control group. After administered with 3μg/kg HS-20001 for db/db mice through single subcutaneous injection, random blood glucose values are decreased a little compared to those of the control group, but no significant difference. The values of random blood glucose of the group of mice administered with liraglutide have no significant decrease.

Table 2: Effect of the administration of the new compounds (mmol/L, $\overline{X}\pm$s, n=8) through single dose on random blood glucose of db / db mice in the same day.

| groups | Before administratio n | Time after administration (h) | | | |
|---|---|---|---|---|---|
| | | 1 | 2 | 4 | 8 |
| control | 25.14±1.09 | 23.66±0.73 | 22.63±0.97 | 22.00±1.00 | 25.39±1.08 |
| Liraglutide -3μg/kg | 25.11+2.33 | 21.78±2.31 | 23.15±2.62 | 21.56±1.37 | 23.93±2.09 |
| HS- 20001-3μg/kg | 25.21±1.44 | 20.34±2.29 | 19.84±1.76 | 20.74±2.51 | 24.29±1.60 |
| HS- 20002-3μg/kg | 25.25±1.57 | 17.86±1.90* | 19.56±0.90* | 18.10±0.79** | 24.19±1.79 |
| HS- 20003-3μg/kg | 25.16±1.49 | 19.56±1.19* | 19.44±1.48 | 19.63±1.12 | 22.59±1.05 |
| HS- 20004-3μg/kg | 25.11±1.63 | 20.11±1.28* | 18.81±1.50* | 17.98±1.38* | 23.30±1.47 |
| HS- 20005-3μg/kg | 25.21±1.56 | 20.11±1.19* | 18.96±1.50* | 18.98±1.48* | 22.36±1.67 |
| HS- 20006-3μg/kg | 25.11±1.49 | 20.36±1.25* | 19.91±1.70* | 19.58±1.54* | 24.30±1.50 |
| HS- 20007-3μg/kg | 25.16±1.63 | 20.43±1.19* | 19.81±1.610* | 20.98±2.38* | 23.42±1.38 |
| HS- 20008-3μg/kg | 25.11±1.58 | 20.56±1.30* | 20.81±1.70* | 19.30±2.02* | 22.41±1.51 |

*P<0.05, **P<0.01, Compared with those of the control group

Table 3: The decrease rate of random blood glucose (%, n=8) of db / db mice administered with the new compounds through single dose in the same day

| group | Time after administration (h) | | | |
|---|---|---|---|---|
| | 1 | 2 | 4 | 8 |
| Liraglutide-3μg/kg | 7.98% | -2.32% | 1.99% | 5.76% |
| HS-20001-3μg/kg | 14.05% | 12.32% | 5.74% | 4.33% |
| HS-20002-3μg/kg | 24.51% | 13.54% | 17.73% | 4.73% |
| HS-20003-3μg/kg | 17.33% | 14.09% | 10.80% | 11.03% |
| HS-20004-3μg/kg | 15.00% | 16.85% | 18.30% | 8.22% |
| HS-20005-3μg/kg | 14.00% | 12.87% | 10.53% | 8.02% |

(continued)

| group | Time after administration (h) | | | |
|---|---|---|---|---|
| | 1 | 2 | 4 | 8 |
| HS-20006-3μg/kg | 14.25% | 13.12% | 10.86% | 8.14% |
| HS-20007-3μg/kg | 13.98% | 11.85% | 9.30% | 6.54% |
| IIS-20008-3μg/kg | 13.90% | 11.62% | 8.90% | 6.25% |

Test 2: Effect of the high-dose new compounds administered by single dose on random blood glucose of db/db mice

The results can be seen in Table 4 and Table 5. db/db mice are administered with 10μg/kg HS-20002 through single subcutaneous injection, after one hour, the random blood glucose values of the mice are decreased significantly compared with those of the control group (P <0.01); after 2h, 4h and 8h from the administration, the random blood glucose values keep a lower level, wherein the values at 4h after administration are most obvious, of which the decrease rate is up to 40.67% and is significantly different from that of the control group (P<0.001), till 24h after the administration, the random blood glucose values are still significantly lower than those of the control group. The mice were administered with 10μg/kg HS-20003 through single subcutaneous injection, after one hour, the random blood glucose values are decreased significantly compared with those of the control group (P <0.01) and is up to 23.62% decreasing, after 2h, 4h and 8h from the administration, the random blood glucose values still keep at a lower level, after 24h from administration, there is no significant difference compared with the control group. db/db mice are administered with 10μg/kg HS-20001 through single subcutaneous injection, after 2h, the random blood glucose values are decreased significantly compared with those of the control group, after 4h and 8h from the administration, the random blood glucose values still keep at a lower level, after 24h from administration, the random blood glucose values show no significant difference from those of the control group. HS-20002, HS-20004, HS-20005, HS-20006, HS-20007 or HS-20008 are administered to mice through single subcutaneous injection and the random blood glucose values are decreased immediately and significantly, the decrease rate is up to 36.20%, 10 after 2h, after 4h and 8h from the administration, the blood glucose values still keep at a lower level, after 24h from the administration, there is no significant difference compared with those of the control group. The values of random blood glucose of mice of group administered with liraglutide have no significant decrease.

Table 4: Effect of the new compounds administered through single dose (mmol/L, ±s, n=8) on the random blood glucose of db / db mice in the same day.

| group | Before administration | Time after administration (h) | | | | |
|---|---|---|---|---|---|---|
| | | 1 | 2 | 4 | 8 | 24 |
| control | 23.08±1.37 | 27.15±1.51 | 28.49±1.58 | 30.76±1.15 | 29.96±0.88 | 27.75±1.64 |
| liraglutide-10μg/kg | 23.19±1.35 | 28.59±1.50 | 28.89±1.17 | 28.55±1.31 | 31.84±0.65 | 27.78±1.14 |
| HS-20001-10μg/kg | 23.16±1.57 | 23.90±1.79 | 20.94±1.57* * | 20.20±1.78* ** | 23.86±1.87* | 24.60±1.92 |
| HS-20002-10μg/kg | 23.15±1.32 | 19.74±1.16* * | 20.31±2.01* * | 18.25±1.98* ** | 22.55±2.20** | 22.60±1.46* |
| HS-20003-10μg/kg | 23.20±1.36 | 20.74±0.98* * | 21.10±0.80* ** | 19.54±1.80* ** | 22.14±2.16** | 24.45±1.55 |
| | | | | | | |
| control | 23.08±1.37 | 30.76±1.15 | 30.29±0.98 | 29.90±0.89 | 31.04±0.94 | 28.98+1 .62 |
| HS-20004-10μg/kg | 23.18±1.65 | 19.63±1.81*** | 21.86±1.66*** | 21.44±1.68** * | 23.80±1.46*** | 25.64±1 .85 |
| HS-20005-10μg/kg | 23.64±1.35 | 19.39±1.61*** | 21.56±1.56*** | 21.49±1.34** * | 23.46±1.51*** | 25.52±1 .68 |
| HS-20006-10μg/kg | 23.54±1.39 | 19.52±1.72*** | 21.43±1.49*** | 21.53±1.67** * | 23.39±1.55*** | 25.59±1 .74 |
| HS-20007-10μg/kg | 23.56±1.42 | 19.41±1.54*** | 21.84±1.57*** | 21.64±1.56** * | 23.81±1.67*** | 25.51+1 .53 |
| HS-20008-10μg/kg | 23.49±1.49 | 19.38±1.83*** | 21.61±1.68*** | 21.72±1.63** * | 23.56±1.80*** | 25.72±1 .69 |

*P<0.05, **P<0.01, ***P<0.001, Compared with the control group

Table 5: The decrease rate of the random blood glucose (%, n=8) of db / db mice administered with the new compounds through single dose in the same day.

| group | Time after administration (h) | | | | |
|---|---|---|---|---|---|
| | 1 | 2 | 4 | 8 | 24 |
| liraglutide-10μg/kg | -5.29% | -1.40% | 7.19% | -6.26% | -0.09% |
| HS-20001-10μg/kg | 11.97% | 26.50% | 34.34% | 20.36% | 11.35% |
| HS-20002-10μg/kg | 27.30% | 28.71% | 40.67% | 24.74% | 18.56% |
| HS-20003-10μg/kg | 23.62% | 25.93% | 36.49% | 26.12% | 11.89% |
| HS-20004-10μg/kg | 36.20% | 27.82% | 28.30% | 23.32% | 11.52% |
| HS-20005-10μg/kg | 37.58% | 28.32% | 29.12% | 24.10% | 12.46% |
| HS-20006-10μg/kg | 38.12% | 27.66% | 29.78% | 23.72% | 13.66% |
| HS-20007-10μg/kg | 36.72% | 25.43% | 26.54% | 23.03% | 12.16% |
| HS-20008-10μg/kg | 35.49% | 25.79% | 27.33% | 22.57% | 14.58% |

Conclusion of the tests:

The random blood glucose of db / db mice administered with series of the new compounds of the invention through single subcutaneous injection can be decreased significantly; the random blood glucose can be decreased obviously by HS-20002, HS-20003, HS-20004, HS-20005, HS-20006, HS-20007 and HS-20008 in a dose of 3μg/kg. Where, HS-20002 and HS-20004 show a much better effect on reducing random blood glucose, the duration of the hypoglycemic effect after single subcutaneous injection is dose-related, the duration of the effect of HS-20002 and HS-20004 on decreasing random blood glucose in the dose of 3μg/kg is more than 4h, and the duration of the effect of HS-20001, HS-20002, HS-20003, HS-20004, HS-20005, HS-20006, HS-20007 and HS-20008 on decreasing random blood glucose in the dose of 10μg/kg is more than 8h.

## Claims

1. A derivative of GLP-1 analog or a pharmaceutically acceptable salt thereof, wherein the amino acid sequence of said GLP-1 analog is selected from:

   His-(D)-Ala-Glu-Gly-Thr-Phe-Thr-Ser-Asp-Leu-Ser-Lys-Gln-Nle-Glu-Glu-Glu-Ala-Val-Arg-Leu-Phe-Ile-Glu-Trp-Leu-Lys-Gln-Gly-Gly-Pro-Ser-Ser-Gly-Ala-Pro-Pro-Pro-Ser-Lys;

   His-(D)-Ala-Glu-Gly-Thr-Phe-Thr-Ser-Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser-Lys (SEQ ID No.3);

   His-Aib-Glu-Gly-Thr-Phe-Thr-Ser-Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser-Lys (SEQ ID No.4);

   His-Ala-Glu-Gly-Thr-Phe-Thr-Ser-Asp-Val-Ser-Ser-Tyr-Leu-Glu-Gly-Gln-Ala-Ala-Glu-Phe-Ile-Val-Trp-Leu-Val-Lys-Gly-Arg-Gly-Lys-Lys-Gly-Gly-Pro-Pro-Pro-Ser-Lys (SEQ ID No.5);

   His-Ala-Glu-Gly-Thr-Phe-Thr-Ser-Asp-Val-Ser-Ser-Tyr-Leu-Glu-Gly-Gln-Ala-Ala-Glu-Phe-Ile-Val-Trp-Leu-Val-Lys-Gly-Arg-Gly-Lys-Lys-Gly-Sar-Pro-Pro-Pro-Ser-Lys (SEQ ID No.6);

   His-Ala-Glu-Gly-Thr-Phe-Thr-Ser-Asp-Val-Ser-Ser-Tyr-Leu-Glu-Glu-Gln-Ala-Ala-Glu-Phe-Ile-Val-Trp-Leu-Val-Lys-Gly-Arg-Gly-Lys-Lys-Gly-Gly-Pro-Pro-Pro-Ser-Lys (SEQ ID No.7); and

His-Ala-Glu-Gly-Thr-Phe-Thr-Ser-Asp-Val-Ser-Ser-Tyr-Leu-Glu-Glu-Gln-Ala-Ala-Glu-Phe-Ile-Val-Trp-Leu-Val-Lys-Gly-Arg-Gly-Lys-Lys-Gly-Sar-Pro-Pro-Pro-Ser-Lys  (SEQ ID No.8),

wherein the derivative of said GLP-1 analog contains a lipophilic substituent of formula $R_1(CH_2)_n$-CO-, in which $R_1$ is selected from $CH_3$- and HOOC-, n is an integer selected from 8-25, wherein the lipophilic substituent of formula $R_1(CH_2)_n$-CO- and the $\alpha$ amino group of the C-terminal Lys of the GLP-1 analog are linked by an amide bond.

2. The derivatives of the GLP-1 analog or a pharmaceutically acceptable salt thereof of claim 1, wherein $R_1$ is $CH_3$-, and n is an integer selected from 8, 10, 12, 14, 16, 18, 20 and 22.

3. The derivatives of the GLP-1 analog or a pharmaceutically acceptable salt thereof of claim 2, wherein $R_1$ is $CH_3$-, and n is 14.

4. The derivatives of the GLP-1 analog or a pharmaceutically acceptable salt thereof of claim 1, wherein $R_1$ is HOOC-, and n is an integer selected from 14, 16, 18, 20 and 22.

5. The derivatives of the GLP-1 analog or a pharmaceutically acceptable salt thereof of claim 4, wherein $R_1$ is HOOC-, and n is 14.

6. The derivative of the GLP-1 analog or a pharmaceutically acceptable salt thereof of claim 1, wherein the amino acid sequence of the GLP-1 analog is SEQ ID NO: 4.

7. A pharmaceutical composition comprising:

   (1) a therapeutically effective amount of the derivatives of the GLP-1 analog or a pharmaceutically acceptable salt thereof of any one of claims 1-6, and
   (2) a pharmaceutically acceptable excipient or drug carrier.

8. Use of the derivatives of the GLP-1 analog or a pharmaceutically acceptable salt thereof of any one of claims 1-6 or the pharmaceutical composition of claim 7 in the preparation of a medicament for the treatment of non-insulin-dependent diabetes mellitus, insulin-dependent diabetes or obesity.

**Patentansprüche**

1. Derivat des GLP-1-Analogons oder ein pharmazeutisch annehmbares Salz hiervon, wobei die Aminosäuresequenz des GLP-1-Analogons gewählt ist aus:

His-(D)-Ala-Glu-Gly-Thr-Phe-Thr-Ser-Asp-Leu-Ser-Lys-Gln-Nle-Glu-Glu-Glu-Ala-Val-Arg-Leu-Phe-Ile-Glu-Trp-Leu-Lys-Gln-Gly-Gly-Pro-Ser-Ser-Gly-Ala-Pro-Pro-Pro-Ser-Lys;

His-(D)-Ala-Glu-Gly-Thr-Phe-Thr-Ser-Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser-Lys (SEQ ID NO: 3);

His-Aib-Glu-Gly-Thr-Phe-Thr-Ser-Asp-Val-Ser-Ser-Tyr-Leu-Glu-Glu-Glu-Ala-Ala-Lys-Glu-Phe-Ile-Ala-Trp-Leu-Val-Arg-Gly-Gly-Pro-Ser-Ser-Gly-Ala-Pro-Pro-Pro-Ser-Lys (SEQ ID NO: 4);

His-Ala-Glu-Gly-Thr-Phe-Thr-Ser-Asp-Val-Ser-Ser-Tyr-Leu-Glu-Gly-Gln-Ala-Ala-Glu-Phe-Ile-Val-Trp-Leu-Val-Lys-Gly-Arg-Gly-Lys-Lys-Gly-Gly-Pro-Pro-Pro-Ser-Lys   (SEQ ID NO: 5);

His-Ala-Glu-Gly-Thr-Phe-Thr-Ser-Asp-Val-Ser-Ser-Tyr-Leu-Glu-Gly-Gln-Ala-Ala-Glu-Phe-Ile-Val-Trp-Leu-Val-Lys-Gly-Arg-Gly-Lys-Lys-Gly-Sar-Pro-Pro-Pro-Ser-Lys   (SEQ ID NO: 6);

His-Ala-Glu-Gly-Thr-Phe-Thr-Ser-Asp-Val-Ser-Ser-Tyr-Leu-Glu-Glu-Gln-Ala-Ala-Glu-Phe-Ile-Val-Trp-Leu-Val-Lys-Gly-Arg-Gly-Lys-Lys-Gly-Gly-Pro-Pro-Pro-Ser-Lys   (SEQ ID NO: 7); und

His-Ala-Glu-Gly-Thr-Phe-Thr-Ser-Asp-Val-Ser-Ser-Tyr-Leu-Glu-Glu-Gln-Ala-Ala-Glu-Phe-Ile-Val-Trp-Leu-Val-Lys-Gly-Arg-Gly-Lys-Lys-Gly-Sar-Pro-Pro-Pro-Ser-Lys   (SEQ ID NO: 8),

wobei das Derivat des GLP-1-Analogons einen lipophilen Substituenten der Formel $R_1(CH_2)_n$-CO- enthält, worin $R_1$ aus $CH_3$- und HOOC- gewählt ist und n eine ganze Zahl gewählt aus 8-25 ist, wobei der lipophile Substituent der Formel $R_1(CH_2)_n$-CO- und die $\alpha$-Aminogruppe des C-terminalen Restes Lys des GLP-1-Analogons über eine Amidbindung miteinander verbunden sind.

2. Derivate des GLP-1-Analogons oder ein pharmazeutisch annehmbares Salz hiervon nach Anspruch 1, wobei $R_1$ $CH_3$- ist und n eine ganze Zahl gewählt aus 8, 10, 12, 14, 16, 18, 20 und 22 ist.

3. Derivate des GLP-1-Analogons oder ein pharmazeutisch annehmbares Salz hiervon nach Anspruch 2, wobei $R_1$ $CH_3$- ist und n 14 ist.

4. Derivate des GLP-1-Analogons oder ein pharmazeutisch annehmbares Salz hiervon nach Anspruch 1, wobei $R_1$ HOOC- ist und n eine ganze Zahl gewählt aus 14, 16, 18, 20 und 22 ist.

5. Derivate des GLP-1-Analogons oder ein pharmazeutisch annehmbares Salz hiervon nach Anspruch 4, wobei $R_1$ HOOC- ist und n 14 ist.

6. Derivat des GLP-1-Analogons oder ein pharmazeutisch annehmbares Salz hiervon nach Anspruch 1, wobei die Aminosäuresequenz des GLP-1-Analogons SEQ ID NO: 4 ist.

7. Pharmazeutische Zusammensetzung, umfassend:

(1) eine therapeutisch wirksame Menge der Derivate des GLP-1-Analogons oder eines pharmazeutisch annehmbaren Salzes hiervon nach einem der Ansprüche 1-6, und
(2) einen pharmazeutisch annehmbaren pharmazeutischen Hilfsstoff oder Wirkstoffträger.

8. Verwendung der Derivate des GLP-1-Analogons oder eines pharmazeutisch annehmbaren Salzes hiervon nach einem der Ansprüche 1-6 oder der pharmazeutischen Zusammensetzung nach Anspruch 7 bei der Herstellung eines Arzneimittels zur Behandlung von Nicht-Insulin-abhängigem Diabetes mellitus, Insulin-abhängigem Diabetes oder Adipositas.

**Revendications**

1. Dérivé d'un analogue de GLP-1 ou un sel pharmaceutiquement acceptable de celui-ci, dans lequel la séquence d'acides aminés dudit analogue de GLP-1 est choisie parmi :

```
His-(D)-Ala-Glu-Gly-Thr-Phe-Thr-Ser-Asp-Leu-Ser-Lys-Gln-
Nle-Glu-Glu-Glu-Ala-Val-Arg-Leu-Phe-Ile-Glu-Trp-Leu-Lys-Gln-
Gly-Gly-Pro-Ser-Ser-Gly-Ala-Pro-Pro-Pro-Ser-Lys ;
```

```
His-(D)-Ala-Glu-Gly-Thr-Phe-Thr-Ser-Asp-Val-Ser-Ser-Tyr-
Leu-Glu-Glu-Glu-Ala-Ala-Lys-Glu-Phe-Ile-Ala-Trp-Leu-Val-Arg-
Gly-Gly-Pro-Ser-Ser-Gly-Ala-Pro-Pro-Pro-Ser-Lys  (SEQ  ID  NO :
3) ;
```

```
His-Aib-Glu-Gly-Thr-Phe-Thr-Ser-Asp-Val-Ser-Ser-Tyr-Leu-
Glu-Glu-Glu-Ala-Ala-Lys-Glu-Phe-Ile-Ala-Trp-Leu-Val-Arg-Gly-
Gly-Pro-Ser-Ser-Gly-Ala-Pro-Pro-Pro-Ser-Lys (SEQ ID NO : 4) ;
```

```
His-Ala-Glu-Gly-Thr-Phe-Thr-Ser-Asp-Val-Ser-Ser-Tyr-Leu-
Glu-Gly-Gln-Ala-Ala-Glu-Phe-Ile-Val-Trp-Leu-Val-Lys-Gly-Arg-
Gly-Lys-Lys-Gly-Gly-Pro-Pro-Pro-Ser-Lys (SEQ ID NO : 5) ;
```

```
His-Ala-Glu-Gly-Thr-Phe-Thr-Ser-Asp-Val-Ser-Ser-Tyr-Leu-
Glu-Gly-Gln-Ala-Ala-Glu-Phe-Ile-Val-Trp-Leu-Val-Lys-Gly-Arg-
Gly-Lys-Lys-Gly-Sar-Pro-Pro-Pro-Ser-Lys (SEQ ID NO : 6) ;
```

```
His-Ala-Glu-Gly-Thr-Phe-Thr-Ser-Asp-Val-Ser-Ser-Tyr-Leu-
Glu-Glu-Gln-Ala-Ala-Glu-Phe-Ile-Val-Trp-Leu-Val-Lys-Gly-Arg-
Gly-Lys-Lys-Gly-Gly-Pro-Pro-Pro-Ser-Lys (SEQ ID NO : 7) ; et
```

```
His-Ala-Glu-Gly-Thr-Phe-Thr-Ser-Asp-Val-Ser-Ser-Tyr-Leu-
Glu-Glu-Gln-Ala-Ala-Glu-Phe-Ile-Val-Trp-Leu-Val-Lys-Gly-Arg-
Gly-Lys-Lys-Gly-Sar-Pro-Pro-Pro-Ser-Lys (SEQ ID NO : 8),
```

dans lequel le dérivé dudit analogue de GLP-1 contient un substituant lipophile de formule $R_1(CH_2)_n$-CO-, dans laquelle $R_1$ est choisi parmi $CH_3$- et HOOC-, n est un nombre entier choisi parmi 8 à 25, dans lequel le substituant lipophile de formule $R_1(CH_2)_n$-CO- et le groupe $\alpha$-aminé de la Lys C-terminale de l'analogue de GLP-1 sont liés par une liaison amide.

2. Dérivés de l'analogue de GLP-1 ou un sel pharmaceutiquement acceptable de ceux-ci selon la revendication 1, dans lesquels $R_1$ est $CH_3$- et n est un nombre entier choisi parmi 8, 10, 12, 14, 16, 18, 20 et 22.

3. Dérivés de l'analogue de GLP-1 ou un sel pharmaceutiquement acceptable de ceux-ci selon la revendication 2,

dans lesquels $R_1$ est $CH_3$- et n est 14.

4. Dérivés de l'analogue de GLP-1 ou un sel pharmaceutiquement acceptable de ceux-ci selon la revendication 1, dans lesquels $R_1$ est HOOC- et n est un nombre entier choisi parmi 14, 16, 18, 20 et 22.

5. Dérivés de l'analogue de GLP-1 ou un sel pharmaceutiquement acceptable de ceux-ci selon la revendication 4, dans lesquels $R_1$ est HOOC- et n est 14.

6. Dérivé de l'analogue de GLP-1 ou un sel pharmaceutiquement acceptable de celui-ci selon la revendication 1, dans lequel la séquence d'acides aminés de l'analogue de GLP-1 est la SEQ ID NO : 4.

7. Composition pharmaceutique comprenant :

(1) une quantité thérapeutiquement efficace des dérivés de l'analogue de GLP-1 ou d'un sel pharmaceutiquement acceptable de ceux-ci selon l'une quelconque des revendications 1 à 6, et
(2) un excipient ou un vecteur de médicament pharmaceutiquement acceptable.

8. Utilisation des dérivés de l'analogue de GLP-1 ou d'un sel pharmaceutiquement acceptable de ceux-ci selon l'une quelconque des revendications 1 à 6 ou de la composition pharmaceutique selon la revendication 7 dans la préparation d'un médicament destiné au traitement du diabète sucré non insulino-dépendant, du diabète insulino-dépendant ou de l'obésité.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9808871 A
- WO 9943705 A
- CN 200680006362
- CN 200680006474
- WO 2007113205 A
- CN 200480004658
- CN 200810152147
- WO 2006097538 A

### Non-patent literature cited in the description

- **CHEN RUIJIE.** Status of research on diabetes drugs. Academic journal of Guangdong College of Pharmacy, 2001, vol. 7, 131-133
- *Endocr Rev.,* 1998, vol. 19 (4), 477-490
- **WEYER C. ; BOGARDUS C. ; MOTT DM. et al.** The natural history of insulin secretory dysfunction and insulin resistance in the pathogenesis of type 2 diabetes mellitus. *J. Clin. Invest.,* 1999, vol. 104 (6), 787-794
- **DRUCKER DJ.** Enhancing incretin action for the treatment of type 2 diabetes. *Diabetes Care.,* 2003, vol. 26 (10), 2929-2940
- **DRUCKER DJ.** Enhancing incretin action for the treatment of type 2 diabetes. *Diabetes Care,* 2003, vol. 26 (10), 2929-2940
- **DEACON CF ; NAUCK MA ; TOFT-NIELSEN M et al.** Both subcutaneously and intravenously administered glucagon-like peptide 1 are rapidly degraded from the NH2-terminus in type 2-diabetic patients and in healthy subjects. *Diabetes,* 1995, vol. 44 (9), 1126-1131
- **GALLWITZ B.** Glucagon-like peptide-1-based therapies for the treatment of type 2 diabetes mellitus. *Treat Endocrinol.,* 2005, vol. 4 (6), 361-370
- **LI Y ; HANSOTIA T ; YUSTA B et al.** Glucagon-like peptide-1 receptor signaling modulates beta cell apoptosis. *J Biol Chem.,* 2003, vol. 278 (1), 471-478
- **DEACON CF.** Therapeutic strategies based on glucagon-like peptide 1. *Diabetes,* 2004, vol. 53 (9), 2181-2189
- **TOFT-NIELSEN MB ; DAMHOLT MB ; MADSBAD S et al.** Determinants of the impaired secretion of glucagon-like peptide-1 in type 2 diabetic patients. *J Clin Endocrinol Metab.,* 2001, vol. 86 (8), 3717-3723
- **DRUCKER DJ.** Nauck MA. The incretin system: glucagon-like peptide-1 receptor agonists and dipeptidyl peptidase-4 inhibitors in type 2 diabetes. *Lancet.,* 2006, vol. 368 (9548), 1696-1705
- **KOLTERMAN OG ; KIM DD ; SHEN L et al.** Pharmacokinetics, pharmacodynamics, and safety of exenatide in patients with type 2 diabetes melllitus. *Am Health Syst Pharm.,* 2005, vol. 62 (2), 173-181
- **NIELSEN LL ; BARON AD.** Pharmacology of exenatide (synthetic exendin-4) for the treatment of type 2 diabetes. *Curr Opin Investig Drugs,* 2003, vol. 4 (4), 401-05
- **KIM JG ; BAGGIO LL ; BRIDON DP et al.** Development and characterization of a glucagon-like peptide-1 albumin conjugate: the ability to activate the glucagon-like peptide 1 receptor in vivo. *Diabetes,* 2003, vol. 52 (3), 751-759
- **BAGGIO LL ; HUANG Q ; BROWN TJ et al.** A Recombinant Human Glucagon-Like Peptide (GLP)-1-Albumin Protein (Albugon) Mimics Peptidergic Activation of GLP-1Receptor-Dependent Pathways Coupled With Satiety, Gastrointestinal Motility, and Glucose Homeostasis. *Diabetes,* 2004, vol. 53 (9), 2492-2500
- *J. Med. Chem.,* 2000, vol. 43, 1664-1669

SEQUENCE LISTING

<110> Jiangsu Hansoh Pharmaceutical Group Co., Ltd.

<120> DERIVATIVE OF A GLP-1 ANALOGUE OR ITS PHARMACEUTICAL SALTS AND THEIR USE

<130> 890086CG

<160> 120

<170> PatentIn version 3.2

<210> 1
<211> 42
<212> PRT
<213> artificial

<220>
<221> misc_feature
<222> (2)..(2)
<223> Xaa represents D-Ala

<400> 1

```
His Xaa Glu Gly Thr Phe Thr Ser Asp Leu Ser Lys Gln Asn Leu Glu
1               5               10                  15

Glu Glu Glu Ala Val Arg Leu Phe Ile Glu Trp Leu Lys Gln Gly Gly
            20              25              30

Pro Ser Ser Gly Ala Pro Pro Pro Ser Lys
        35              40
```

<210> 2
<211> 40
<212> PRT
<213> artificial

<400> 2

```
His Gly Glu Gly Thr Phe Thr Ser Asp Leu Ser Lys Gln Met Glu Glu
1               5               10                  15

Glu Ala Val Arg Leu Phe Ile Glu Trp Leu Lys Asn Gly Gly Pro Ser
            20              25              30

Ser Gly Ala Pro Pro Pro Ser Lys
        35              40
```

<210> 3
<211> 40
<212> PRT
<213> artificial

<220>
<221> misc_feature
<222> (2)..(2)
<223> Xaa represents D-Ala

<400> 3

```
His Xaa Glu Gly Thr Phe Thr Ser Asp Val Ser Ser Tyr Leu Glu Glu
1               5               10                  15

Glu Ala Ala Lys Glu Phe Ile Ala Trp Leu Val Arg Gly Gly Pro Ser
            20              25              30

Ser Gly Ala Pro Pro Pro Ser Lys
        35              40
```

<210> 4
<211> 40
<212> PRT
<213> artificial

<220>

<220>
<221> misc_feature
<222> (2)..(2)
<223> Xaa represents Aib

<400> 4

```
His Xaa Glu Gly Thr Phe Thr Ser Asp Val Ser Ser Tyr Leu Glu Glu
1               5               10                  15

Glu Ala Ala Lys Glu Phe Ile Ala Trp Leu Val Arg Gly Gly Pro Ser
            20              25                  30

Ser Gly Ala Pro Pro Pro Ser Lys
            35              40
```

<210> 5
<211> 39
<212> PRT
<213> artificial

<400> 5

```
His Ala Glu Gly Thr Phe Thr Ser Asp Val Ser Ser Tyr Leu Glu Gly
1               5               10                  15

Gln Ala Ala Glu Phe Ile Val Trp Leu Val Lys Gly Arg Gly Lys Lys
            20              25                  30

Gly Gly Pro Pro Pro Ser Lys
            35
```

<210> 6
<211> 39
<212> PRT
<213> artificial

<220>
<221> misc_feature
<222> (34)..(34)
<223> Xaa represents Sar

<400> 6

```
His Ala Glu Gly Thr Phe Thr Ser Asp Val Ser Ser Tyr Leu Glu Gly
1               5               10                  15

Gln Ala Ala Glu Phe Ile Val Trp Leu Val Lys Gly Arg Gly Lys Lys
            20              25                  30

Gly Xaa Pro Pro Pro Ser Lys
            35
```

<210> 7
<211> 39
<212> PRT
<213> artificial

<400> 7

```
        His Ala Glu Gly Thr Phe Thr Ser Asp Val Ser Ser Tyr Leu Glu Glu
        1               5                   10                  15

        Gln Ala Ala Glu Phe Ile Val Trp Leu Val Lys Gly Arg Gly Lys Lys
                    20                  25                  30

        Gly Gly Pro Pro Pro Ser Lys
                    35
```

<210> 8
<211> 39
<212> PRT
<213> artificial

<220>
<221> misc_feature
<222> (34)..(34)
<223> Xaa represents Sar

<400> 8

```
        His Ala Glu Gly Thr Phe Thr Ser Asp Val Ser Ser Tyr Leu Glu Glu
        1               5                   10                  15

        Gln Ala Ala Glu Phe Ile Val Trp Leu Val Lys Gly Arg Gly Lys Lys
                    20                  25                  30

        Gly Xaa Pro Pro Pro Ser Lys
                    35
```

<210> 9
<211> 39
<212> PRT
<213> artificial

<220>
<221> misc_feature
<222> (16)..(16)
<223> Xaa represents Aib

<400> 9

```
His Ala Glu Gly Thr Phe Thr Ser Asp Val Ser Ser Tyr Leu Glu Xaa
1               5               10              15

Gln Ala Ala Glu Phe Ile Val Trp Leu Val Lys Gly Arg Gly Lys Lys
            20              25              30

Gly Gly Pro Pro Pro Ser Lys
            35
```

<210> 10
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 10

```
His Ala Glu Gly Thr Phe Thr Ser Asp Val Ser Ser Tyr Leu Glu Xaa
1               5               10              15

Gln Ala Ala Glu Phe Ile Val Trp Leu Val Lys Gly Arg Gly Lys Lys
            20              25              30

Gly Xaa Pro Pro Pro Ser Lys
            35
```

<210> 11
<211> 39
<212> PRT
<213> artificial

<400> 11

```
His Ala Glu Gly Thr Phe Thr Ser Asp Val Ser Ser Tyr Met Glu Gly
1               5               10              15

Gln Ala Ala Glu Phe Ile Val Trp Leu Val Lys Gly Arg Gly Lys Lys
                20              25              30

Gly Gly Pro Pro Pro Ser Lys
            35
```

<210> 12
<211> 39
<212> PRT
<213> artificial

<400> 12

His Ala Glu Gly Thr Phe Thr Ser Asp Val Ser Ser Tyr Met Glu Glu
1               5                   10                  15

Gln Ala Ala Glu Phe Ile Val Trp Leu Val Lys Gly Arg Gly Lys Lys
                20              25                  30

Gly Gly Pro Pro Pro Ser Lys
            35

<210> 13
<211> 39
<212> PRT
<213> artificial

<220>
<221> misc_feature
<222> (34)..(34)
<223> Xaa represents Sar

<400> 13

His Ala Glu Gly Thr Phe Thr Ser Asp Val Ser Ser Tyr Met Glu Glu
1               5                   10                  15

Gln Ala Ala Glu Phe Ile Val Trp Leu Val Lys Gly Arg Gly Lys Lys
                20              25                  30

Gly Xaa Pro Pro Pro Ser Lys
            35

<210> 14
<211> 39
<212> PRT
<213> artificial

<220>
<221> misc_feature
<222> (16)..(16)
<223> Xaa represents Aib

<400> 14

His Ala Glu Gly Thr Phe Thr Ser Asp Val Ser Ser Tyr Met Glu Xaa

|   | 1 | | | | 5 | | | | | 10 | | | | | 15 |
|---|---|---|---|---|---|---|---|---|---|---|---|---|---|---|---|

Gln Ala Ala Glu Phe Ile Val Trp Leu Val Lys Gly Arg Gly Lys Lys
　　　　　20　　　　　　　　25　　　　　　　30

Gly Gly Pro Pro Pro Ser Lys
　　　　35

<210> 15
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 15

His Ala Glu Gly Thr Phe Thr Ser Asp Val Ser Ser Tyr Met Glu Xaa
1　　　　　　5　　　　　　　　10　　　　　　　15

Gln Ala Ala Glu Phe Ile Val Trp Leu Val Lys Gly Arg Gly Lys Lys
　　　　　20　　　　　　　　25　　　　　　　30

Gly Xaa Pro Pro Pro Ser Lys
　　　　35

<210> 16
<211> 39
<212> PRT
<213> artificial

<400> 16

His Ala Glu Gly Thr Phe Thr Ser Asp Val Ser Ser Gln Leu Glu Gly
1　　　　　　5　　　　　　　　10　　　　　　　15

Gln Ala Ala Glu Phe Ile Val Trp Leu Val Lys Gly Arg Gly Lys Lys
　　　　　20　　　　　　　　25　　　　　　　30

Gly Gly Pro Pro Pro Ser Lys
　　　　35

<210> 17
<211> 39

<212> PRT
<213> artificial

<220>
<221> misc_feature
<222> (34)..(34)
<223> Xaa represents Sar

<400> 17

```
His Ala Glu Gly Thr Phe Thr Ser Asp Val Ser Ser Gln Leu Glu Gly
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 18
<211> 39
<212> PRT
<213> artificial

<400> 18

```
His Ala Glu Gly Thr Phe Thr Ser Asp Val Ser Ser Gln Leu Glu Glu
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 19
<211> 39
<212> PRT
<213> artificial

<220>
<221> misc_feature
<222> (34)..(34)
<223> Xaa represents Sar

<400> 19

His Ala Glu Gly Thr Phe Thr Ser Asp Val Ser Ser Gln Leu Glu Glu
1               5               10              15

Gln Ala Ala Glu Phe Ile Val Trp Leu Val Lys Gly Arg Gly Lys Lys
              20              25              30

Gly Xaa Pro Pro Pro Ser Lys
        35

<210> 20
<211> 39
<212> PRT
<213> artificial

<220>
<221> misc_feature
<222> (16)..(16)
<223> Xaa represents Aib

<400> 20

His Ala Glu Gly Thr Phe Thr Ser Asp Val Ser Ser Gln Leu Glu Xaa
1               5               10              15

Gln Ala Ala Glu Phe Ile Val Trp Leu Val Lys Gly Arg Gly Lys Lys
              20              25              30

Gly Gly Pro Pro Pro Ser Lys
        35

<210> 21
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 21

```
His Ala Glu Gly Thr Phe Thr Ser Asp Val Ser Ser Gln Leu Glu Xaa
1               5               10              15
```

```
Gln Ala Ala Glu Phe Ile Val Trp Leu Val Lys Gly Arg Gly Lys Lys
            20              25              30
```

```
Gly Xaa Pro Pro Pro Ser Lys
        35
```

<210> 22
<211> 39
<212> PRT
<213> artificial

<400> 22

```
His Ala Glu Gly Thr Phe Thr Ser Asp Val Ser Ser Gln Met Glu Gly
1               5               10              15
```

```
Gln Ala Ala Glu Phe Ile Val Trp Leu Val Lys Gly Arg Gly Lys Lys
            20              25              30
```

```
Gly Gly Pro Pro Pro Ser Lys
        35
```

<210> 23
<211> 39
<212> PRT
<213> artificial

<220>
<221> misc_feature
<222> (34)..(34)
<223> Xaa represents Sar

<400> 23

```
His Ala Glu Gly Thr Phe Thr Ser Asp Val Ser Ser Gln Met Glu Gly
1               5               10              15
```

```
Gln Ala Ala Glu Phe Ile Val Trp Leu Val Lys Gly Arg Gly Lys Lys
            20              25              30
```

```
Gly Xaa Pro Pro Pro Ser Lys
        35
```

<210> 24
<211> 39
<212> PRT
<213> artificial

<400> 24

```
His Ala Glu Gly Thr Phe Thr Ser Asp Val Ser Ser Gln Met Glu Glu
1               5               10                  15

Gln Ala Ala Glu Phe Ile Val Trp Leu Val Lys Gly Arg Gly Lys Lys
            20              25                  30

Gly Gly Pro Pro Pro Ser Lys
            35
```

<210> 25
<211> 39
<212> PRT
<213> artificial

<220>
<221> misc_feature
<222> (34)..(34)
<223> Xaa represents Sar

<400> 25

```
His Ala Glu Gly Thr Phe Thr Ser Asp Val Ser Ser Gln Met Glu Glu
1               5               10                  15

Gln Ala Ala Glu Phe Ile Val Trp Leu Val Lys Gly Arg Gly Lys Lys
            20              25                  30

Gly Xaa Pro Pro Pro Ser Lys
            35
```

<210> 26
<211> 39
<212> PRT
<213> artificial

<220>
<221> misc_feature
<222> (16)..(16)
<223> Xaa represents Aib

<400> 26

```
His Ala Glu Gly Thr Phe Thr Ser Asp Val Ser Ser Gln Met Glu Xaa
1               5               10                  15

Gln Ala Ala Glu Phe Ile Val Trp Leu Val Lys Gly Arg Gly Lys Lys
            20              25                  30

Gly Gly Pro Pro Pro Ser Lys
            35
```

<210> 27

<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 27

```
His Ala Glu Gly Thr Phe Thr Ser Asp Val Ser Ser Gln Met Glu Xaa
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 28
<211> 39
<212> PRT
<213> artificial

<400> 28

```
His Ala Glu Gly Thr Phe Thr Ser Asp Val Ser Lys Tyr Leu Glu Gly
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 29
<211> 39
<212> PRT
<213> artificial

<220>
<221> misc_feature
<222> (34)..(34)
<223> Xaa represents Sar

<400> 29

```
His Ala Glu Gly Thr Phe Thr Ser Asp Val Ser Lys Tyr Leu Glu Gly
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 30
<211> 39
<212> PRT
<213> artificial

<400> 30

```
His Ala Glu Gly Thr Phe Thr Ser Asp Val Ser Lys Tyr Leu Glu Glu
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 31
<211> 39
<212> PRT
<213> artificial

<220>
<221> misc_feature
<222> (34)..(34)
<223> Xaa represents Sar

<400> 31

```
His Ala Glu Gly Thr Phe Thr Ser Asp Val Ser Lys Tyr Leu Glu Glu
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 32
<211> 39
<212> PRT
<213> artificial

<220>

<221> misc_feature
<222> (16)..(16)
<223> Xaa represents Aib

<400> 32

```
His Ala Glu Gly Thr Phe Thr Ser Asp Val Ser Lys Tyr Leu Glu Xaa
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 33
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 33

```
His Ala Glu Gly Thr Phe Thr Ser Asp Val Ser Lys Tyr Leu Glu Xaa
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 34
<211> 39
<212> PRT
<213> artificial

<400> 34

```
        His Ala Glu Gly Thr Phe Thr Ser Asp Val Ser Lys Tyr Met Glu Gly
        1                   5                   10                  15


        Gln Ala Ala Glu Phe Ile Val Trp Leu Val Lys Gly Arg Gly Lys Lys
                        20                  25                  30


        Gly Gly Pro Pro Pro Ser Lys
                        35
```

<210> 35
<211> 39
<212> PRT
<213> artificial

<220>
<221> misc_feature
<222> (34)..(34)
<223> Xaa represents Sar

<400> 35

```
        His Ala Glu Gly Thr Phe Thr Ser Asp Val Ser Lys Tyr Met Glu Gly
        1                   5                   10                  15


        Gln Ala Ala Glu Phe Ile Val Trp Leu Val Lys Gly Arg Gly Lys Lys
                        20                  25                  30


        Gly Xaa Pro Pro Pro Ser Lys
                        35
```

<210> 36
<211> 39
<212> PRT
<213> artificial

<400> 36

```
        His Ala Glu Gly Thr Phe Thr Ser Asp Val Ser Lys Tyr Met Glu Glu
        1                   5                   10                  15


        Gln Ala Ala Glu Phe Ile Val Trp Leu Val Lys Gly Arg Gly Lys Lys
                        20                  25                  30


        Gly Gly Pro Pro Pro Ser Lys
                        35
```

<210> 37
<211> 39
<212> PRT
<213> artificial

<220>

<221> misc_feature
<222> (34)..(34)
<223> Xaa represents Sar

<400> 37

His Ala Glu Gly Thr Phe Thr Ser Asp Val Ser Lys Tyr Met Glu Glu
1               5               10              15

Gln Ala Ala Glu Phe Ile Val Trp Leu Val Lys Gly Arg Gly Lys Lys
        20              25              30

Gly Xaa Pro Pro Pro Ser Lys
        35

<210> 38
<211> 39
<212> PRT
<213> artificial

<220>
<221> misc_feature
<222> (16)..(16)
<223> Xaa represents Aib

<400> 38

His Ala Glu Gly Thr Phe Thr Ser Asp Val Ser Lys Tyr Met Glu Xaa
1               5               10              15

Gln Ala Ala Glu Phe Ile Val Trp Leu Val Lys Gly Arg Gly Lys Lys
        20              25              30

Gly Gly Pro Pro Pro Ser Lys
        35

<210> 39
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 39

```
His Ala Glu Gly Thr Phe Thr Ser Asp Val Ser Lys Tyr Met Glu Xaa
1               5                   10                  15

Gln Ala Ala Glu Phe Ile Val Trp Leu Val Lys Gly Arg Gly Lys Lys
                20                  25                  30

Gly Xaa Pro Pro Pro Ser Lys
        35
```

<210> 40
<211> 39
<212> PRT
<213> artificial

<400> 40

```
His Ala Glu Gly Thr Phe Thr Ser Asp Val Ser Lys Gln Leu Glu Gly
1               5                   10                  15

Gln Ala Ala Glu Phe Ile Val Trp Leu Val Lys Gly Arg Gly Lys Lys
                20                  25                  30

Gly Gly Pro Pro Pro Ser Lys
        35
```

<210> 41
<211> 39
<212> PRT
<213> artificial

<220>
<221> misc_feature
<222> (34)..(34)
<223> Xaa represents Sar

<400> 41

```
His Ala Glu Gly Thr Phe Thr Ser Asp Val Ser Lys Gln Leu Glu Gly
1               5                   10                  15

Gln Ala Ala Glu Phe Ile Val Trp Leu Val Lys Gly Arg Gly Lys Lys

                20                  25                  30

Gly Xaa Pro Pro Pro Ser Lys
        35
```

<210> 42
<211> 39
<212> PRT
<213> artificial

<400> 42

```
His Ala Glu Gly Thr Phe Thr Ser Asp Val Ser Lys Gln Leu Glu Glu
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 43
<211> 39
<212> PRT
<213> artificial

<220>
<221> misc_feature
<222> (34)..(34)
<223> Xaa represents Sar

<400> 43

```
His Ala Glu Gly Thr Phe Thr Ser Asp Val Ser Lys Gln Leu Glu Glu
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 44
<211> 39
<212> PRT
<213> artificial

<220>
<221> misc_feature
<222> (16)..(16)
<223> Xaa represents Aib

<400> 44

```
His Ala Glu Gly Thr Phe Thr Ser Asp Val Ser Lys Gln Leu Glu Xaa
```

|    | 1     |     |     | 5     |     |     |     | 10    |     |     |     |     | 15  |
|----|-------|-----|-----|-------|-----|-----|-----|-------|-----|-----|-----|-----|-----|

Gln Ala Ala Glu Phe Ile Val Trp Leu Val Lys Gly Arg Gly Lys Lys
            20                    25                30

Gly Gly Pro Pro Pro Ser Lys
            35

<210> 45
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 45

His Ala Glu Gly Thr Phe Thr Ser Asp Val Ser Lys Gln Leu Glu Xaa
1               5                   10                  15

Gln Ala Ala Glu Phe Ile Val Trp Leu Val Lys Gly Arg Gly Lys Lys
            20                    25                30

Gly Xaa Pro Pro Pro Ser Lys
            35

<210> 46
<211> 39
<212> PRT
<213> artificial

<400> 46

His Ala Glu Gly Thr Phe Thr Ser Asp Val Ser Lys Gln Met Glu Gly
1               5                   10                  15

Gln Ala Ala Glu Phe Ile Val Trp Leu Val Lys Gly Arg Gly Lys Lys
            20                    25                30

Gly Gly Pro Pro Pro Ser Lys
            35

<210> 47
<211> 39

<212> PRT
<213> artificial

<220>
<221> misc_feature
<222> (34)..(34)
<223> Xaa represents Sar

<400> 47

```
His Ala Glu Gly Thr Phe Thr Ser Asp Val Ser Lys Gln Met Glu Gly
1               5                   10                  15

Gln Ala Ala Glu Phe Ile Val Trp Leu Val Lys Gly Arg Gly Lys Lys
                20                  25                  30

Gly Xaa Pro Pro Pro Ser Lys
        35
```

<210> 48
<211> 39
<212> PRT
<213> artificial

<400> 48

```
His Ala Glu Gly Thr Phe Thr Ser Asp Val Ser Lys Gln Met Glu Glu
1               5                   10                  15

Gln Ala Ala Glu Phe Ile Val Trp Leu Val Lys Gly Arg Gly Lys Lys
                20                  25                  30

Gly Gly Pro Pro Pro Ser Lys
        35
```

<210> 49
<211> 39
<212> PRT
<213> artificial

<220>
<221> misc_feature
<222> (34)..(34)
<223> Xaa represents Sar

<400> 49

```
        His Ala Glu Gly Thr Phe Thr Ser Asp Val Ser Lys Gln Met Glu Glu
        1               5               10              15
```

```
        Gln Ala Ala Glu Phe Ile Val Trp Leu Val Lys Gly Arg Gly Lys Lys
                    20              25              30
```

```
        Gly Xaa Pro Pro Pro Ser Lys
                35
```

<210> 50
<211> 39
<212> PRT
<213> artificial

<220>
<221> misc_feature
<222> (16)..(16)
<223> Xaa represents Aib

<400> 50

```
        His Ala Glu Gly Thr Phe Thr Ser Asp Val Ser Lys Gln Met Glu Xaa
        1               5               10              15
```

```
        Gln Ala Ala Glu Phe Ile Val Trp Leu Val Lys Gly Arg Gly Lys Lys
                    20              25              30
```

```
        Gly Gly Pro Pro Pro Ser Lys
                35
```

<210> 51
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 51

```
His Ala Glu Gly Thr Phe Thr Ser Asp Val Ser Lys Gln Met Glu Xaa
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 52
<211> 39
<212> PRT
<213> artificial

<400> 52

```
His Ala Glu Gly Thr Phe Thr Ser Asp Leu Ser Lys Gln Leu Glu Gly
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 53
<211> 39
<212> PRT
<213> artificial

<220>
<221> misc_feature
<222> (34)..(34)
<223> Xaa represents Sar

<400> 53

```
His Ala Glu Gly Thr Phe Thr Ser Asp Leu Ser Lys Gln Leu Glu Gly
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 54
<211> 39
<212> PRT
<213> artificial

<400> 54

```
His Ala Glu Gly Thr Phe Thr Ser Asp Leu Ser Lys Gln Leu Glu Glu
1               5               10              15

Gln Ala Ala Glu Phe Ile Val Trp Leu Val Lys Gly Arg Gly Lys Lys
            20              25              30

Gly Gly Pro Pro Pro Ser Lys
            35
```

<210> 55
<211> 39
<212> PRT
<213> artificial

<220>
<221> misc_feature
<222> (34)..(34)
<223> Xaa represents Sar

<400> 55

```
His Ala Glu Gly Thr Phe Thr Ser Asp Leu Ser Lys Gln Leu Glu Glu
1               5               10              15

Gln Ala Ala Glu Phe Ile Val Trp Leu Val Lys Gly Arg Gly Lys Lys
            20              25              30

Gly Xaa Pro Pro Pro Ser Lys
            35
```

<210> 56
<211> 39
<212> PRT
<213> artificial

<220>
<221> misc_feature
<222> (16)..(16)
<223> Xaa represents Aib

<400> 56

```
His Ala Glu Gly Thr Phe Thr Ser Asp Leu Ser Lys Gln Leu Glu Xaa
1               5               10              15

Gln Ala Ala Glu Phe Ile Val Trp Leu Val Lys Gly Arg Gly Lys Lys
            20              25              30

Gly Gly Pro Pro Pro Ser Lys
            35
```

<210> 57

<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 57

```
His Ala Glu Gly Thr Phe Thr Ser Asp Leu Ser Lys Gln Leu Glu Xaa
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 58
<211> 39
<212> PRT
<213> artificial

<400> 58

```
His Ala Glu Gly Thr Phe Thr Ser Asp Leu Ser Lys Gln Met Glu Gly
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 59
<211> 39
<212> PRT
<213> artificial

<220>
<221> misc_feature
<222> (34)..(34)
<223> Xaa represents Sar

<400> 59

```
His Ala Glu Gly Thr Phe Thr Ser Asp Leu Ser Lys Gln Met Glu Gly
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 60
<211> 39
<212> PRT
<213> artificial

<400> 60

```
His Ala Glu Gly Thr Phe Thr Ser Asp Leu Ser Lys Tyr Leu Glu Gly
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 61
<211> 39
<212> PRT
<213> artificial

<220>
<221> misc_feature
<222> (34)..(34)
<223> Xaa represents Sar

<400> 61

```
His Ala Glu Gly Thr Phe Thr Ser Asp Leu Ser Lys Tyr Leu Glu Gly
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 62
<211> 39
<212> PRT
<213> artificial

<400> 62

```
His Ala Glu Gly Thr Phe Thr Ser Asp Leu Ser Lys Tyr Met Glu Gly
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 63
<211> 39
<212> PRT
<213> artificial

<220>
<221> misc_feature
<222> (34)..(34)
<223> Xaa represents Sar

<400> 63

```
His Ala Glu Gly Thr Phe Thr Ser Asp Leu Ser Lys Tyr Met Glu Gly
1               5               10              15

Gln Ala Ala Glu Phe Ile Val Trp Leu Val Lys Gly Arg Gly Lys Lys
            20              25              30

            Gly Xaa Pro Pro Pro Ser Lys
                35
```

<210> 64
<211> 39
<212> PRT
<213> artificial

<400> 64

```
His Ala Glu Gly Thr Phe Thr Ser Asp Leu Ser Ser Gln Leu Glu Gly
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 65
<211> 39
<212> PRT
<213> artificial

<220>
<221> misc_feature

<222> (34)..(34)
<223> Xaa represents Sar

<400> 65

```
His Ala Glu Gly Thr Phe Thr Ser Asp Leu Ser Ser Gln Leu Glu Gly
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 66
<211> 39
<212> PRT
<213> artificial

<400> 66

```
His Ala Glu Gly Thr Phe Thr Ser Asp Leu Ser Ser Gln Met Glu Gly
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 67
<211> 39
<212> PRT
<213> artificial

<220>
<221> misc_feature
<222> (34)..(34)
<223> Xaa represents Sar

<400> 67

```
His Ala Glu Gly Thr Phe Thr Ser Asp Leu Ser Ser Gln Met Glu Gly
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 68

<211> 39
<212> PRT
<213> artificial

<400> 68

```
His Ala Glu Gly Thr Phe Thr Ser Asp Leu Ser Ser Tyr Leu Glu Gly
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 69
<211> 39
<212> PRT
<213> artificial

<220>
<221> misc_feature
<222> (34)..(34)
<223> Xaa represents Sar

<400> 69

```
His Ala Glu Gly Thr Phe Thr Ser Asp Leu Ser Ser Tyr Leu Glu Gly
1               5                   10                  15

Gln Ala Ala Glu Phe Ile Val Trp Leu Val Lys Gly Arg Gly Lys Lys
            20                  25                  30

        Gly Xaa Pro Pro Pro Ser Lys
                35
```

<210> 70
<211> 39
<212> PRT
<213> artificial

<400> 70

```
His Ala Glu Gly Thr Phe Thr Ser Asp Leu Ser Ser Tyr Met Glu Gly
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 71

<211> 39
<212> PRT
<213> artificial

<220>
<221> misc_feature
<222> (34)..(34)
<223> Xaa represents Sar

<400> 71

```
His Ala Glu Gly Thr Phe Thr Ser Asp Leu Ser Ser Tyr Met Glu Gly
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 72
<211> 39
<212> PRT
<213> artificial

<400> 72

```
His Lys Glu Gly Thr Phe Thr Ser Asp Val Ser Ser Tyr Leu Glu Gly
1               5                   10                  15

Gln Ala Ala Glu Phe Ile Val Trp Leu Val Lys Gly Arg Gly Lys Lys
            20                  25                  30

            Gly Gly Pro Pro Pro Ser Lys
                35
```

<210> 73
<211> 39
<212> PRT
<213> artificial

<220>
<221> misc_feature
<222> (34)..(34)
<223> Xaa represents Sar

<400> 73

```
His Lys Glu Gly Thr Phe Thr Ser Asp Val Ser Ser Tyr Leu Glu Gly
1               5               10              15

Gln Ala Ala Glu Phe Ile Val Trp Leu Val Lys Gly Arg Gly Lys Lys
                20              25              30

Gly Xaa Pro Pro Pro Ser Lys
        35
```

<210> 74
<211> 39
<212> PRT
<213> artificial

<400> 74

```
His Lys Glu Gly Thr Phe Thr Ser Asp Val Ser Ser Tyr Leu Glu Glu
1               5               10              15

Gln Ala Ala Glu Phe Ile Val Trp Leu Val Lys Gly Arg Gly Lys Lys
                20              25              30

Gly Gly Pro Pro Pro Ser Lys
        35
```

<210> 75
<211> 39
<212> PRT
<213> artificial

<220>
<221> misc_feature
<222> (34)..(34)
<223> Xaa represents Sar

<400> 75

```
His Leu Glu Gly Thr Phe Thr Ser Asp Val Ser Ser Tyr Leu Glu Glu
1               5               10              15

Gln Ala Ala Glu Phe Ile Val Trp Leu Val Lys Gly Arg Gly Lys Lys
                20              25              30

Gly Xaa Pro Pro Pro Ser Lys
        35
```

<210> 76
<211> 39
<212> PRT
<213> artificial

<220>

<221> misc_feature
<222> (16)..(16)
<223> Xaa represents Aib

<400> 76

```
His Leu Glu Gly Thr Phe Thr Ser Asp Val Ser Ser Tyr Leu Glu Xaa
1               5                   10                  15

Gln Ala Ala Glu Phe Ile Val Trp Leu Val Lys Gly Arg Gly Lys Lys
                20                  25                  30

Gly Gly Pro Pro Pro Ser Lys
            35
```

<210> 77
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 77

```
His Lys Glu Gly Thr Phe Thr Ser Asp Val Ser Ser Tyr Leu Glu Xaa
1               5                   10                  15

Gln Ala Ala Glu Phe Ile Val Trp Leu Val Lys Gly Arg Gly Lys Lys
                20                  25                  30

Gly Xaa Pro Pro Pro Ser Lys
            35
```

<210> 78
<211> 39
<212> PRT
<213> artificial

<400> 78

```
His Lys Glu Gly Thr Phe Thr Ser Asp Val Ser Ser Tyr Met Glu Gly
1               5               10              15

Gln Ala Ala Glu Phe Ile Val Trp Leu Val Lys Gly Arg Gly Lys Lys
            20              25              30

Gly Gly Pro Pro Pro Ser Lys
            35
```

<210> 79
<211> 39
<212> PRT
<213> artificial

<220>
<221> misc_feature
<222> (34)..(34)
<223> Xaa represents Sar

<400> 79

```
His Ile Glu Gly Thr Phe Thr Ser Asp Val Ser Ser Tyr Met Glu Gly
1               5               10              15

Gln Ala Ala Glu Phe Ile Val Trp Leu Val Lys Gly Arg Gly Lys Lys
            20              25              30

Gly Xaa Pro Pro Pro Ser Lys
            35
```

<210> 80
<211> 39
<212> PRT
<213> artificial

<400> 80

```
His Ile Glu Gly Thr Phe Thr Ser Asp Val Ser Ser Tyr Met Glu Glu
1               5               10              15

Gln Ala Ala Glu Phe Ile Val Trp Leu Val Lys Gly Arg Gly Lys Lys
            20              25              30

Gly Gly Pro Pro Pro Ser Lys
            35
```

<210> 81
<211> 39
<212> PRT
<213> artificial

<220>

<221> misc_feature
<222> (34)..(34)
<223> Xaa represents Sar

<400> 81

```
His Ile Glu Gly Thr Phe Thr Ser Asp Val Ser Ser Tyr Met Glu Glu
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 82
<211> 39
<212> PRT
<213> artificial

<220>
<221> misc_feature
<222> (16)..(16)
<223> Xaa represents Aib

<400> 82

```
His Leu Glu Gly Thr Phe Thr Ser Asp Val Ser Ser Tyr Met Glu Xaa
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 83
<211> 39
<212> PRT
<213> artificial

<220>
<221> misc_feature
<222> (2)..(2)
<223> Xaa represents Aib

<220>
<221> misc_feature
<222> (34)..(34)
<223> Xaa represents Sar

<400> 83

```
His Xaa Glu Gly Thr Phe Thr Ser Asp Leu Ser Ser Tyr Met Glu Gly


1               5               10              15


Gln Ala Ala Glu Phe Ile Val Trp Leu Val Lys Gly Arg Gly Lys Lys
            20              25              30


Gly Xaa Pro Pro Pro Ser Lys
        35
```

<210> 84
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 84

```
His Lys Glu Gly Thr Phe Thr Ser Asp Val Ser Ser Tyr Met Glu Xaa
1               5               10              15


Gln Ala Ala Glu Phe Ile Val Trp Leu Val Lys Gly Arg Gly Lys Lys
            20              25              30


Gly Xaa Pro Pro Pro Ser Lys
        35
```

<210> 85
<211> 39
<212> PRT
<213> artificial

<400> 85

```
His Ile Glu Gly Thr Phe Thr Ser Asp Val Ser Ser Gln Leu Glu Gly
1               5               10              15


Gln Ala Ala Glu Phe Ile Val Trp Leu Val Lys Gly Arg Gly Lys Lys
            20              25              30


Gly Gly Pro Pro Pro Ser Lys
        35
```

<210> 86
<211> 39
<212> PRT
<213> artificial

<220>
<221> misc_feature
<222> (34)..(34)
<223> Xaa represents Sar

<400> 86

```
His Val Glu Gly Thr Phe Thr Ser Asp Val Ser Ser Gln Leu Glu Gly
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 87
<211> 39
<212> PRT
<213> artificial

<400> 87

```
His Val Glu Gly Thr Phe Thr Ser Asp Val Ser Ser Gln Leu Glu Glu
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 88
<211> 39
<212> PRT
<213> artificial

<220>
<221> misc_feature
<222> (34)..(34)
<223> Xaa represents Sar

<400> 88

```
His Val Glu Gly Thr Phe Thr Ser Asp Val Ser Ser Gln Leu Glu Glu
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 89
<211> 39
<212> PRT
<213> artificial

<220>
<221> misc_feature
<222> (16)..(16)
<223> Xaa represents Aib

<400> 89

```
His Leu Glu Gly Thr Phe Thr Ser Asp Val Ser Ser Gln Leu Glu Xaa
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 90
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 90

```
His Lys Glu Gly Thr Phe Thr Ser Asp Val Ser Ser Gln Leu Glu Xaa
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 91
<211> 39
<212> PRT
<213> artificial

<400> 91

```
His Lys Glu Gly Thr Phe Thr Ser Asp Val Ser Ser Gln Met Glu Gly
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 92
<211> 39
<212> PRT
<213> artificial

<220>
<221> misc_feature
<222> (34)..(34)
<223> Xaa represents Sar

<400> 92

```
His Val Glu Gly Thr Phe Thr Ser Asp Val Ser Ser Gln Met Glu Gly
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 93
<211> 39
<212> PRT
<213> artificial

<400> 93

```
        His Val Glu Gly Thr Phe Thr Ser Asp Val Ser Ser Gln Met Glu Glu
        1               5               10              15


        Gln Ala Ala Glu Phe Ile Val Trp Leu Val Lys Gly Arg Gly Lys Lys
                    20              25              30


        Gly Gly Pro Pro Pro Ser Lys
                    35
```

<210> 94
<211> 39
<212> PRT
<213> artificial

<220>
<221> misc_feature
<222> (34)..(34)
<223> Xaa represents Sar

<400> 94

```
        His Gly Glu Gly Thr Phe Thr Ser Asp Val Ser Ser Gln Met Glu Glu
        1               5               10              15


        Gln Ala Ala Glu Phe Ile Val Trp Leu Val Lys Gly Arg Gly Lys Lys
                    20              25              30


        Gly Xaa Pro Pro Pro Ser Lys
                    35
```

<210> 95
<211> 39
<212> PRT
<213> artificial

<220>
<221> misc_feature
<222> (16)..(16)
<223> Xaa represents Aib

<400> 95

```
        His Gly Glu Gly Thr Phe Thr Ser Asp Val Ser Ser Gln Met Glu Xaa
        1               5               10              15


        Gln Ala Ala Glu Phe Ile Val Trp Leu Val Lys Gly Arg Gly Lys Lys
                    20              25              30


        Gly Gly Pro Pro Pro Ser Lys
                    35
```

<210> 96

<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 96

```
His Ile Glu Gly Thr Phe Thr Ser Asp Val Ser Ser Gln Met Glu Xaa
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 97
<211> 39
<212> PRT
<213> artificial

<400> 97

```
His Ile Glu Gly Thr Phe Thr Ser Asp Val Ser Lys Tyr Leu Glu Gly
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 98
<211> 39
<212> PRT
<213> artificial

<220>
<221> misc_feature
<222> (34)..(34)
<223> Xaa represents Sar

<400> 98

```
        His Lys Glu Gly Thr Phe Thr Ser Asp Val Ser Lys Tyr Leu Glu Gly
        1               5               10              15

        Gln Ala Ala Glu Phe Ile Val Trp Leu Val Lys Gly Arg Gly Lys Lys
                    20              25              30

        Gly Xaa Pro Pro Pro Ser Lys
                    35
```

<210> 99
<211> 39
<212> PRT
<213> artificial

<400> 99

```
        His Lys Glu Gly Thr Phe Thr Ser Asp Val Ser Lys Tyr Leu Glu Glu
        1               5               10              15

        Gln Ala Ala Glu Phe Ile Val Trp Leu Val Lys Gly Arg Gly Lys Lys
                    20              25              30

        Gly Gly Pro Pro Pro Ser Lys
                    35
```

<210> 100
<211> 39
<212> PRT
<213> artificial

<220>
<221> misc_feature
<222> (34)..(34)
<223> Xaa represents Sar

<400> 100

```
        His Ile Glu Gly Thr Phe Thr Ser Asp Val Ser Lys Tyr Leu Glu Glu
        1               5               10              15

        Gln Ala Ala Glu Phe Ile Val Trp Leu Val Lys Gly Arg Gly Lys Lys
                    20              25              30

        Gly Xaa Pro Pro Pro Ser Lys
                    35
```

<210> 101
<211> 39
<212> PRT
<213> artificial

<220>

<221> misc_feature
<222> (16)..(16)
<223> Xaa represents Aib

<400> 101

```
His Val Glu Gly Thr Phe Thr Ser Asp Val Ser Lys Tyr Leu Glu Xaa
1               5                   10                  15

Gln Ala Ala Glu Phe Ile Val Trp Leu Val Lys Gly Arg Gly Lys Lys
            20                  25                  30

Gly Gly Pro Pro Pro Ser Lys
            35
```

<210> 102
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 102

```
His Val Glu Gly Thr Phe Thr Ser Asp Val Ser Lys Tyr Leu Glu Xaa
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
            35
```

<210> 103
<211> 39
<212> PRT
<213> artificial

<400> 103

```
His Val Glu Gly Thr Phe Thr Ser Asp Val Ser Lys Tyr Met Glu Gly
1               5               10              15

Gln Ala Ala Glu Phe Ile Val Trp Leu Val Lys Gly Arg Gly Lys Lys
            20              25              30

Gly Gly Pro Pro Pro Ser Lys
            35
```

<210> 104
<211> 39
<212> PRT
<213> artificial

<220>
<221> misc_feature
<222> (34)..(34)
<223> Xaa represents Sar

<400> 104

```
His Ile Glu Gly Thr Phe Thr Ser Asp Val Ser Lys Tyr Met Glu Gly
1               5               10              15

Gln Ala Ala Glu Phe Ile Val Trp Leu Val Lys Gly Arg Gly Lys Lys
            20              25              30

Gly Xaa Pro Pro Pro Ser Lys
            35
```

<210> 105
<211> 39
<212> PRT
<213> artificial

<400> 105

```
His Lys Glu Gly Thr Phe Thr Ser Asp Val Ser Lys Tyr Met Glu Glu
1               5               10              15

Gln Ala Ala Glu Phe Ile Val Trp Leu Val Lys Gly Arg Gly Lys Lys
            20              25              30

Gly Gly Pro Pro Pro Ser Lys
            35
```

<210> 106
<211> 39
<212> PRT
<213> artificial

<220>

<221> misc_feature
<222> (34)..(34)
<223> Xaa represents Sar

<400> 106

```
His Ile Glu Gly Thr Phe Thr Ser Asp Val Ser Lys Tyr Met Glu Glu
1               5                   10                  15

Gln Ala Ala Glu Phe Ile Val Trp Leu Val Lys Gly Arg Gly Lys Lys
            20                  25                  30

Gly Xaa Pro Pro Pro Ser Lys
        35
```

<210> 107
<211> 39
<212> PRT
<213> artificial

<220>
<221> misc_feature
<222> (16)..(16)
<223> Xaa represents Aib

<400> 107

```
His Lys Glu Gly Thr Phe Thr Ser Asp Val Ser Lys Tyr Met Glu Xaa
1               5                   10                  15

Gln Ala Ala Glu Phe Ile Val Trp Leu Val Lys Gly Arg Gly Lys Lys
            20                  25                  30

Gly Gly Pro Pro Pro Ser Lys
        35
```

<210> 108
<211> 39
<212> PRT
<213> artificial

<220>
<221> misc_feature
<222> (16)..(16)
<223> Xaa represents Aib

<220>
<221> misc_feature
<222> (34)..(34)
<223> Xaa represents Sar

<400> 108

```
        His Val Glu Gly Thr Phe Thr Ser Asp Val Ser Lys Tyr Met Glu Xaa
        1               5               10              15
```

```
        Gln Ala Ala Glu Phe Ile Val Trp Leu Val Lys Gly Arg Gly Lys Lys
                    20              25              30
```

```
        Gly Xaa Pro Pro Ser Lys
                35
```

<210> 109
<211> 39
<212> PRT
<213> artificial

<400> 109

```
        His Lys Glu Gly Thr Phe Thr Ser Asp Val Ser Lys Gln Leu Glu Gly
        1               5               10              15
```

```
        Gln Ala Ala Glu Phe Ile Val Trp Leu Val Lys Gly Arg Gly Lys Lys
                    20              25              30
```

```
        Gly Gly Pro Pro Pro Ser Lys
                35
```

<210> 110
<211> 39
<212> PRT
<213> artificial

<220>
<221> misc_feature
<222> (34)..(34)
<223> Xaa represents Sar

<400> 110

```
        His Ile Glu Gly Thr Phe Thr Ser Asp Val Ser Lys Gln Leu Glu Gly
        1               5               10              15
```

```
        Gln Ala Ala Glu Phe Ile Val Trp Leu Val Lys Gly Arg Gly Lys Lys
                        20              25              30
```

```
          Gly Xaa Pro Pro Pro Ser Lys
                  35
```

<210> 111
<211> 39
<212> PRT
<213> artificial

EP 2 460 825 B1

<400> 111

```
His Lys Glu Gly Thr Phe Thr Ser Asp Val Ser Lys Gln Leu Glu Glu
1               5               10              15

Gln Ala Ala Glu Phe Ile Val Trp Leu Val Lys Gly Arg Gly Lys Lys
            20              25              30

Gly Gly Pro Pro Pro Ser Lys
        35
```

<210> 112
<211> 39
<212> PRT
<213> artificial

<220>
<221> misc_feature
<222> (2)..(2)
<223> Xaa represents Aib

<220>
<221> misc_feature
<222> (34)..(34)
<223> Xaa represents Sar

<400> 112

```
His Xaa Glu Gly Thr Phe Thr Ser Asp Val Ser Lys Gln Leu Glu Glu
1               5               10              15

Gln Ala Ala Glu Phe Ile Val Trp Leu Val Lys Gly Arg Gly Lys Lys
            20              25              30

Gly Xaa Pro Pro Pro Ser Lys
        35
```

<210> 113
<211> 39
<212> PRT
<213> artificial

<220>
<221> misc_feature
<222> (16)..(16)
<223> Xaa represents Aib

<400> 113

**67**

```
His Lys Glu Gly Thr Phe Thr Ser Asp Val Ser Lys Gln Leu Glu Xaa
1               5               10              15

Gln Ala Ala Glu Phe Ile Val Trp Leu Val Lys Gly Arg Gly Lys Lys
        20              25              30

Gly Gly Pro Pro Pro Ser Lys
        35
```

<210> 114
<211> 39
<212> PRT
<213> artificial

<220>
<221> misc_feature
<222> (2)..(2)
<223> Xaa represents Aib

<220>
<221> misc_feature
<222> (16)..(16)
<223> Xaa represents Aib

<220>
<221> misc_feature
<222> (34)..(34)
<223> Xaa represents Sar

<400> 114

```
His Xaa Glu Gly Thr Phe Thr Ser Asp Val Ser Lys Gln Leu Glu Xaa
1               5               10              15

Gln Ala Ala Glu Phe Ile Val Trp Leu Val Lys Gly Arg Gly Lys Lys
        20              25              30

Gly Xaa Pro Pro Pro Ser Lys
        35
```

<210> 115
<211> 39
<212> PRT
<213> artificial

<400> 115

```
His Ile Glu Gly Thr Phe Thr Ser Asp Val Ser Lys Gln Met Glu Gly
1               5               10              15

Gln Ala Ala Glu Phe Ile Val Trp Leu Val Lys Gly Arg Gly Lys Lys
        20              25              30
```

```
                    Gly Gly Pro Pro Pro Ser Lys
                                35
```

<210> 116
<211> 39
<212> PRT
<213> artificial

<220>
<221> misc_feature
<222> (2)..(2)
<223> Xaa represents Aib

<220>
<221> misc_feature
<222> (34)..(34)
<223> Xaa represents Sar

<400> 116

```
        His Xaa Glu Gly Thr Phe Thr Ser Asp Val Ser Lys Gln Met Glu Gly
        1               5                   10                  15

        Gln Ala Ala Glu Phe Ile Val Trp Leu Val Lys Gly Arg Gly Lys Lys
                    20                  25                  30

        Gly Xaa Pro Pro Pro Ser Lys
                    35
```

<210> 117
<211> 39
<212> PRT
<213> artificial

<400> 117

```
        His Gly Glu Gly Thr Phe Thr Ser Asp Val Ser Lys Gln Met Glu Glu
        1               5                   10                  15

        Gln Ala Ala Glu Phe Ile Val Trp Leu Val Lys Gly Arg Gly Lys Lys
                    20                  25                  30

        Gly Gly Pro Pro Pro Ser Lys
                    35
```

<210> 118
<211> 39
<212> PRT
<213> artificial

<220>
<221> misc_feature
<222> (2)..(2)
<223> Xaa represents Aib

<220>
<221> misc_feature
<222> (34)..(34)
<223> Xaa represents Sar

<400> 118

```
His Xaa Glu Gly Thr Phe Thr Ser Asp Val Ser Lys Gln Met Glu Glu
1               5                   10                  15

Gln Ala Ala Glu Phe Ile Val Trp Leu Val Lys Gly Arg Gly Lys Lys
                20                  25                  30

Gly Xaa Pro Pro Pro Ser Lys
        35
```

<210> 119
<211> 39
<212> PRT
<213> artificial

<220>
<221> misc_feature
<222> (16)..(16)
<223> Xaa represents Aib

<400> 119

```
His Lys Glu Gly Thr Phe Thr Ser Asp Val Ser Lys Gln Met Glu Xaa
1               5                   10                  15

Gln Ala Ala Glu Phe Ile Val Trp Leu Val Lys Gly Arg Gly Lys Lys
                20                  25                  30

Gly Gly Pro Pro Pro Ser Lys
        35
```

<210> 120
<211> 39
<212> PRT
<213> artificial

<220>
<221> misc_feature
<222> (2)..(2)
<223> Xaa represents Aib

<220>
<221> misc_feature
<222> (16)..(16)
<223> Xaa represents Aib

<220>
<221> misc_feature

<222> (34)..(34)
<223> Xaa represents Sar

<400> 120

```
His Xaa Glu Gly Thr Phe Thr Ser Asp Val Ser Lys Gln Met Glu Xaa
1               5               10              15

Gln Ala Ala Glu Phe Ile Val Trp Leu Val Lys Gly Arg Gly Lys Lys
        20              25              30

Gly Xaa Pro Pro Pro Ser Lys
        35
```